# EUROPEAN PATENT SPECIFICATION

(11) **EP 4 161 418 B1**
(45) Date of publication and mention of the grant of the patent: **24.12.2025**
(21) Application number: 21732547.1
(22) Date of filing: 07.06.2021
(51) Int. Cl.: A61B 18/12, A61B 18/14, A61B 18/00

(54) **ABLATION EQUIPMENT TO TREAT TARGET REGIONS OF TISSUE IN ORGANS**
ABLATIONSVORRICHTUNG ZUR BEHANDLUNG VON ZIELGEWEBEREGIONEN IN ORGANEN
ÉQUIPEMENT D'ABLATION PERMETTANT DE TRAITER DES RÉGIONS CIBLES DE TISSU DANS DES ORGANES

(30) Priority: 07.06.2020 US 202063035807 P
(43) Date of publication of application: 12.04.2023
(73) Proprietor: ARGA' MEDTECH SA, 1066 Epalinges (CH)
(72) Inventor: WERNETH, Randell L., San Diego, California 92127 (US); ZARBATANY, David, Laguna Niguel, California 92677 (US); ROMAN, Ricardo David, Chula Vista, California 91911 (US); SHERMAN, Marshall, Cardiff, CA 92007 (US)
(74) Representative: Eisenführ Speiser
(86) International application number: PCT/IB2021/054965
(87) International publication number: WO 2021/250538

(56) References cited:
- US-A- 5 882 346
- US-A1- 2010 152 725
- US-A1- 2012 220 998
- US-A1- 2014 066 913
- US-A1- 2019 151 008

## Description

### . Field of the invention

**.** The present invention relates to ablation equipment or ablation assemblies to treat target regions of tissue in organs systems and methods for treating target regions of tissue in organs.

**.** More particularly, the present invention relates to a combination system and method for non-thermally treating target tissue and thermally ablating tissue. Said tissue would be that which is either diseased such as in atrial fibrillation (or AF) patient where the cardiac cell action potential is not normal, typically phase phases 0-3. Said tissue could also be tissue where it is deemed necessary to block a refractory wave-front to stop or prevent irregular arrhythmias in patients.

**.** The present invention relates generally to ablation systems and methods for performing targeted tissue ablation in a patient. In particular, the present invention provides catheters which deliver RadioFrequency (RF) and/or IRreversible Electroporation (IRE) which occurs when a strong, Pulsed Electrical Field (PEF) causes permeabilization of the cell membrane, leading to cellular homeostasis disruption and cell death. Irreversible Electroporation (IRE) energies that create safe, precision lesions in targeted tissue such as that cause heart arrhythmias.

### . Background art

**.** Applications of PEF in cardiology are vast and include atrial fibrillation, ventricular fibrillation, septal ablation, and targeting vascular structures. PEF has appealing characteristics including ability to be tissue specific and non-thermal. This invention provides for a novel catheter design to delivery IRE / PEF to cardiac tissue.

**.** Pulsed electric fields (PEF) refer to application of intermittent, high-intensity electric fields for short periods of time (micro- or nanoseconds), which results in cellular and tissue electroporation. Electroporation is a process whereby an applied electric field (i.e. PEF) results in formation of pores in cell membranes. Pore formation leads to permeabilization, which can be reversible or irreversible, depending upon parameters of the applied PEF. In reversible electroporation, cells remain viable., and underlies the basis of electrochemotherapy and gene electrotransfer. See references 1) Mali B, Jarm T, Snoj M, Sersa G, Miklavcic D. Antitumor effectiveness of electrochemotherapy: A systematic review and meta-analysis. Eur J Surg Oncol. 2013;39:4-16; 2) Heller R, Heller LC. Gene Electrotransfer Clinical Trials. Adv Genet. 2015;89:235-62; 3) Neumann E, Schaefer-Ridder M, Wang Y, Hofschneider P. Gene transfer into mouse lyoma cells by electroporation in high electric fields. EMBO J. 1982;1:841-5.

**.** Electroporation is a phenomenon whereby PEF (created by high voltage currents) are applied to a cell resulting in pore formation in the cell membrane with a subsequent increase in cell permeability. The electric field is most commonly produced by high voltage direct current delivered between two or more electrodes. When electric fields are applied, charge is established across the lipid bilayer and, once a critical threshold is reached (dependent on transmembrane voltage), electroporation occurs. In contrast, with irreversible electroporation (IRE), cells and tissue are non-viable because of programmed cell death cascade activation. IRE is a wellestablished treatment for solid tumors. However, PEFs may also be useful in cardiology, particularly for cardiac ablation, given limitations of current thermal based approaches. PEF can create lesions without tissue heating, and be cell/tissue selective which enables preservation of critical surrounding structures.

**.** Tissue ablation is used in numerous medical procedures to treat a patient. Ablation can be performed to remove or denature undesired tissue such as diseased cardiac cells. Ablation procedures may also involve the modification of the tissue without removal, such as to stop electrical function in a particular area in the chain of electrical propagation through the heart tissue in patients with an arrhythmia condition. The ablation can be performed by passing energy, such as electrical energy, through one or more electrodes and causing tissue death where the electrodes are in contact. Ablation procedures can be performed on patients with any cardiac arrhythmia such as atrial fibrillation (AF) by ablating tissue in the heart.

**.** Mammalian organ function typically occurs when electrical activity is spontaneously generated by the SA node, the cardiac pacemaker. This electrical impulse is propagated throughout the right atrium, and through Bachmann's bundle to the left atrium, stimulating the myocardium of the atria to contract. The conduction system consists of specialized heart muscle cells. Cardiac myocardial cell has a negative membrane potential when at rest. Stimulation above a threshold value induces the opening of voltage-gated ion channels and a flood of cations into the cell. The positively charged ions entering the cell cause the depolarization characteristic of an action potential. Like skeletal muscle, depolarization causes the opening of voltage-gated calcium channels and release of Ca2+ from the t-tubules. This influx of calcium causes calcium-induced calcium release from the sarcoplasmic reticulum, and free Ca2+ causes muscle contraction. After a delay, potassium channels reopen, and the resulting flow of K+ out of the cell causes repolarization to the resting state. This transmission of electrical impulses propagates through the heart chamber. A disturbance of such electrical transmission may lead to organ malfunction. One particular area where electrical impulse transmission is critical for proper organ function is in the heart, resulting in atrial contractions which leads to the pumping of blood into the ventricles in a manner synchronous with the pulse.

**.** Atrial fibrillation (AF) refers to a type of cardiac arrhythmia where there is disorganized electrical conduction in the atria causing rapid uncoordinated atrial contractions that result in ineffective pumping of blood into the ventricle as well as a lack of synchrony. During AF, the atrioventricular node receives electrical impulses from numerous locations throughout the atria instead of only from the sinus node. These aberrant signals overwhelm the atrioventricular node, producing an irregular and rapid heartbeat. As a result, blood may pool in the atria, increasing the likelihood of blood clot, hypertension, diabetes, and thyrotoxicosis. AF affects 7% of the population over age 65.

**.** Atrial fibrillation treatment options are limited. Lifestyle changes only assist individuals with lifestyle related AF. Medication therapy manages AF symptoms, often presents side effects more dangerous than AF, and fails to cure AF. Electrical cardioversion attempts to restore a normal sinus rhythm, but has a high AF recurrence rate due to disease progression. In addition, if there is a blood clot in the atria, cardioversion may cause the clot to leave the heart and travel to the brain (causing a stroke) or to some other part of the body. What are needed are new methods for treating AF and other medical conditions involving disorganized electrical conduction.

**.** Various ablation techniques have been proposed to treat AF, including the Cox-Maze ablation procedure, linear ablation of various regions of the atrium, and circumferential ablation of pulmonary vein ostia. The Cox-Maze ablation procedure and linear ablation procedures are tedious and time-consuming, taking several hours to accomplish. Current pulmonary vein ostial ablation is proving to be ineffective long-term. All ablation procedures involve the risk of inadvertently damaging untargeted tissue, such as the esophagus while ablating tissue in the left atrium of the heart. There is therefore a need for improved atrial ablation products and techniques that create efficacious lesions in a safe manner.

**.** Applications of non-thermal and thermal ablation in cardiology are vast and include treating patients with atrial fibrillation, ventricular fibrillation, septal ablation, and vascular structures diseases. Ablation has appealing characteristics including ability to be tissues.

**.** Cardiac ablation technology for medical treatment is known in the art and includes such treatment modalities as radiofrequency (RF), focused ultrasound, such as high intensity ultrasound beams, microwave, laser, thermal electric heating, traditional heating methods with electrodes using direct current (DC) or alternating current (AC), and application of heated fluids and cold therapies (such as cryosurgery, also known as cryotherapy or cryoablation).

**.** Solutions are known in the following documents: US8641704B2, US8475449B2, US2010152725A1, US2010152725A1, US8948865B2, US2008281314A1, US8540710B2, US2019038171A1, US8221411B2, US2016051324A1, US2015327994A1, WO2017192804A1, US2020229866A1, WO2019023280A1.

**.** In many of these procedures an energy delivery device, such as a probe with or without a needle, is inserted into a target tissue to cause destruction of a target region of the cardiac tissue through the application of energy, such as thermal energy, non-thermal energy, and energy associated with cryo ablation procedures. An elongated catheter or access tube is typically used to create the means to deliver the ablation elements into the heart.

**.** Once in place, the tissue immediately adjacent to the energy delivery device or electrodes is ablated. This can produce a focalized zone around the ablation elements, maximizing the chance of death in the desired tissue location. It is known in the art that electrically induced thermal ablation such as RF can be used to effectively and continuously locally ablate a tissue site as an energy delivery device is placed on the tissue surface. RF can lead to coagulation necrosis in a margin surrounding normal tissue where hyperthermic conditions lead to cellular injury such as coagulation of cytosolic enzymes and damage to histone complexes, leading to ultimate cell death. Although these tissue treatment methods and systems can effectively ablate volumes of target tissue, there are limitations to each technique. One often cited problem using these procedures during cardiac ablation involves heat sink, a process whereby one aspect can include blood flow whereas the heat generated on the ablation element will be removed/dissipated by the cooler blood flows over the element. This heat dissipation effect can change both the shape and maximum volume of the tissue being ablated. After treatment of a target tissue region with an energy delivery device, upon removal of the energy delivery device from the targeted tissue region, the energy delivery device can be placed in a new, un-ablated site needing treatment.

**.** More recently, irreversible electroporation (IRE) has been used as an alternative to the above-mentioned procedures to ablate cardiac or organ tissue. However, though IRE can be a non-thermal method causing cell death, it is not ideal for coagulation, and specifically does not cause electrically induced thermal coagulation, demonstrating the importance of using an alternative source such as RF or long DC pulses in heating a tissue site. Instead, IRE involves the application of electrical pulses to targeted tissue in the range of microseconds to milliseconds that can lead to non-thermally produced defects in the cell membrane that are nanoscale in size. These defects can lead to a disruption of homeostasis of the cell membrane, thereby causing irreversible cell membrane permeabilization which induces cell necrosis, without raising the temperature of the tissue ablation zone. During IRE ablation, connective tissue and scaffolding structures are spared, thus allowing the surrounding organs, structures, blood vessels, and connective tissue to remain intact. With nonthermal IRE (hereinafter also called non-thermal IRE), cell death is mediated through a nonthermal mechanism, so the heat sink problem associated with many ablation techniques is nullified. Therefore the advantages of IRE to allow focused treatment with tissue sparing and without thermal effects can be used effectively in conjunction with thermal treatment such as RF that has been proven effective to prevent ablation site bleeding; this will also allow (in this example embodiment) the user to utilize determined RF levels leading to in some cases ablation and in some cases coagulation; this is important since IRE will not effectively coagulate when dealing with large tissue regions. In this way the newly discovered advantages of IRE can be utilized effectively with known techniques of nonthermal damage with the added advantage of either selecting to use RF or no RF in conjunction.

**.** Although IRE has distinct advantages, there are also advantages of utilizing thermal ablation during treatment procedures. Prior to the disclosure of this invention, an invention had not been proposed that could solve the problems of nonthermally ablating a target region of cardiac or organ tissue, while maintaining integrity of the surrounding tissue, and effectively switching to a device for effectively thermally ablating tissue along the ablation track. In certain proposed embodiments, an energy delivery device can be utilized that is powered by a single energy source that is capable of application of energy in various forms, and subsequently ablating a tissue track during a medical procedure for the treatment of arrhythmias using the same energy delivery device that can be powered by a different form of energy from the same energy source, to maximize procedure outcomes.

**.** More recently, IRreversible Electroporation (IRE) has been used as a means to ablate cardiac tissue or organ tissue. However, though IRE can be a non-thermal method causing cell death, traditional delivery is with a Direct Current (DC) or a Square-wave pulse.

. Since square wave voltage signals cause significant cardiac muscle stimulation, they are not ideal for ensuring the overall safety of the subject having the ablation procedure.

. Therefore, the use of an alternative solution for delivering both non-thermal and thermal energy would be highly desirable and safer for patients.

. US patent application US 2010/0152725 A1 relates to a system for selectively ablating tissue that has at least one energy source that has a non-thermal energy source and a thermal energy source, at least one probe, means for selectively coupling the probe to one desired energy source of the at least one energy source, means for selectively energizing the non-thermal energy source of the at least one energy source to apply nonthermal energy to at least a portion of the desired region to ablate at least a portion of the desired region, and means for selectively energizing the thermal energy source of the at least one energy source during the withdrawal of the at least one probe to thermally ablate tissue substantially adjacent to a probe track.

### . Solution

. This invention provides for a novel assembly or equipment for delivering non-thermal and thermal energies to cardiac tissue. All references to methods below are not part of the claimed invention but are useful for the general understanding of the invention.

. It is a purpose of this invention, in certain embodiments, to provide a combination treatment system that has at least one energy delivery device, or ablation catheter 1, and at least one power or energy or power source, or single power source 4, that is capable of providing IRE energy and thermal energy to the energy delivery device. The at least one energy delivery device can be either a monopolar or bipolar device. The single power source 4 electrically powers the at least one energy delivery device through an electric signal comprising a sinusoidal wave to deliver both non-thermal energy for treating a tissue and thermal energy for ablating the tissue. The system can continuously modify the energy or power source from energy utilized in a nonthermal form to energy in a thermal form to ablate target regions of tissue as well as tissue along a track.

**.** It is a further purpose of this invention to provide a method that involves using non-thermal IRE energy and thermal energy to effectively ablate target regions of tissue. The method involves positioning at least one energy delivery device that is coupled to a single power source within a target region of a tissue, applying IRE energy from the power source to the energy delivery device which is used to ablate a target region of tissue, while preventing damage to surrounding structures, then switching from IRE energy to thermal energy using the same power source, and positioning the energy delivery device while ablating said tissue with thermal energy such as RF energy, to allow for focal tissue ablation and the safe energy delivery used during the treatment procedure, while among other things, coagulating tissue and preventing bleeding.

**.** What is described herein is a system 3 and method for selectively ablating tissue, the system 3 comprising an ablation catheter 1 and a single power source 4.

**.** According to alternative embodiments, the method involves providing application of IRE to ablate and or treat tissue and treatment of tissue with an alternative energy form (such as thermal energy) to effectively ablate tissue from the same ablation device and the same energy source. The method can involve providing at least one energy source, or single power source 4, which has at least a non-thermal energy source 6 and a thermal energy source 7, providing at least one probe, or ablation catheter 1, that is configured to be selectively operatively coupled to a desired energy source of the at least one energy source, positioning via a probe at least a portion of the at least one probe within a desired region of a heart or organ, selectively coupling the at least one probe to the non-thermal energy source, selectively energizing the non-thermal energy source to apply nonthermal energy from the non-thermal energy source to at least a portion of the desired region to ablate at least a portion of the desired region, selectively coupling the at least one probe to the thermal energy source, withdrawing the at least probe from the desired region, and selectively energizing the thermal energy source to apply thermal energy during at least a portion of withdrawal of the at least one probe to ablate tissue substantially adjacent to the probe track.

**.** According to alternative embodiments, a system for selectively ablating tissue 3 is provided herein that has at least one energy source, or single power source 4, that has a non-thermal energy source 6 and a thermal energy source 7, at least one probe, or ablation catheter 1, a means for selectively coupling 8 the probe to one desired energy source of the at least one energy source means for selectively energizing the non-thermal energy source 11 of the at least one energy source to apply non-thermal energy to at least a portion of the desired region to ablate at least a portion of the desired region, and means for selectively energizing the thermal energy source 12 of the at least one energy source during the withdrawal of the at least one probe to thermally ablate tissue substantially adjacent to a probe track.

**.** According to alternative embodiments, a unique multi-electrode and multi-functional ablation catheter and ablation catheter systems, or ablation assembly or equipment 100, and methods are provided which map and ablate myocardial tissue within the heart chambers of a patient. Any electrocardiogram signal site (e.g. a site with aberrant signals) or combination of multiple sites that are discovered with this placement may be ablated. In alternative embodiments, the ablation catheters and systems may be used to treat non-cardiac patient tissue, such as tumor tissue, renal artery nerves, etc.

**.** According to alternative embodiments, a probe, e.g. an ablation catheter 1 for performing a medical procedure on a patient is provided. The ablation catheter 1 comprises an elongate shaft 13 with a proximal portion 14 including a proximal end 15 and a distal end 16, and a distal portion 17 with a proximal end 18 and a distal end 19. The elongate shaft 13 further comprises a shaft ablation assembly 20 and a distal ablation assembly 21 configured to deliver energy, such as RF and/or Electroporation energy, to tissue 41. The shaft ablation assembly 20 is proximal to the distal end of the distal portion 19, and includes at least one shaft ablation element 22, or shaft electrode 127, fixedly or removable attached to the shaft 13 and configured to deliver ablation energy to tissue. The distal ablation assembly 21 is at the distal end of the distal portion 19 and includes at least one tip ablation element 23, or electrode tip 128, configured to deliver ablation energy to tissue 41.

**.** According to alternative embodiments, the distal portion 17 is configured to be in a circular configuration and can be deflected in one or more directions, in one or more deflection shapes and geometries 24. The deflection geometries 24 may be similar or symmetric deflection geometries, or the deflection geometries may be dissimilar or asymmetric deflection geometries. The shaft, or ablation catheter 1, may include one or more steering wires 25 configured to deflect the distal portion 17 in the one or more deflection directions. The catheter deflection can also occur by placing or removing a shape setting mandrel 26 within a center lumen in the catheter. The elongate shaft 13 may include difference is the stiffness of the shaft along its length. The elongate shaft 13 may include a shape setting mandrel 26 within the shaft, or ablation catheter 1, the shape setting mandrel 26 configured to perform or enhance the deflection (steering and shape) of the distal portion 17, such as to maintain deflections in a single plane. The shaft, or ablation catheter, may include variable material properties such as a asymmetric joint 27 between two portions, an integral member 28 within a wall or fixedly attached to the shaft, a variable braid 29, or other variation used to create multiple deflections, such as deflections with asymmetric deflection geometries.

**.** According to alternative embodiments, the distal ablation assembly 21 may be fixedly attached to the distal end of the distal portion 19, or it may be advanced from the distal shaft 17, such as via a port 30. The distal ablation assembly 21 may comprise a single ablation element 31, such as an electrode, or tip ablation element 23 or electrode tip 128, or multiple ablation elements 32, such as electrodes, or mandrel electrodes 132. The distal ablation assembly 21 may include a shape setting mandrel carrier assembly 33 of ablation elements, or simply shape setting mandrel 26, and the shape setting mandrel carrier assembly 33 may be changeable from a compact geometry to an expanded geometry, such transition caused by advancement and/or retraction of a control shaft or the mandrel.

**.** According to alternative embodiments, the shaft ablation assembly 20 may include a single ablation element 31 or multiple ablation elements 32, or shaft electrodes 127, preferably five to ten ablation elements fixedly attached to the shaft or shape setting mandrel. The ablation elements may have a profile that is flush with the surface of the shaft, or more preferably the shaft between the electrode elements outer diameter 35, or shaft outer diameter 35, is slightly smaller than the diameter of the ablation electrodes 36, or shaft electrodes outer diameter 36, such that the distal end of the catheter is more flexible.

**.** According to alternative embodiments, the ablation elements 31, 32, 127, 128, 132 of the present invention can deliver one or more forms of energy, preferably RF and/or High Voltages Electroporation energy. The ablation elements may have similar or dissimilar construction, and may be constructed in various sizes and geometries. The ablation elements may include one or more thermocouples 37, such as two thermocouples mounted 90º from each other on the inside of an ablation element. The ablation elements may include means of dissipating heat 38, such as increased surface area. According to alternative embodiments, one or more ablation elements is configured in a tubular geometry, and the wall thickness to outer diameter approximates a 1:15 ratio. According to alternative embodiments, one or more ablation elements is configured to record, or map electrical activity in tissue such as mapping of cardiac electrograms. According to alternative embodiments, one or more ablation elements is configured to deliver pacing energy, such as to energy delivered to pace the heart of a patient.

**.** According to alternative embodiments, the ablation catheters of the present invention may be used to treat one or more medical conditions by delivering ablation energy to tissue. Conditions include an arrhythmia of the heart, cancer, and other conditions in which removing or denaturing tissue improves the patient's health.

**.** According to alternative embodiments, a method of treating atrial flutter is provided. An ablation catheter of the present invention may be used to achieve bi-directional block, such as by placement in one or more locations in the right atrium of the heart 43.

**.** According to alternative embodiments, a method of ablating tissue in the right atrium of the heart is provided. An ablation catheter of the present invention may be used to: create lesions between the superior vena cava and the inferior vena cava; the coronary sinus and the inferior vena cava; the superior vena cava and the coronary sinus; and combinations of these. The catheter can be used to map electrograms and/or map and/or ablate the sinus node, such as to treat sinus node tachycardia.

**.** According to alternative embodiments, a method of treating ventricular tachycardia is provided. An ablation catheter of the present invention may be placed in the left or right ventricles of the heart, induce ventricular tachycardia by delivering pacing energy, and ablating tissue to treat the patient.

**.** According to alternative embodiments, an ablation catheter with a first geometry larger than a second deflection geometry is provided via the shape setting mandrel. The ablation catheter is placed in the smaller second shape geometry to ablate one or more of the following tissue locations: left atrial septum; tissue adjacent the left atrial septum; and tissue adjacent the left atrial posterior wall. The ablation catheter is placed in the larger first geometry to ablate at least the circumference around the pulmonary veins.

**.** According to alternative embodiments, an ablation catheter of the present invention is used to treat both the left and right atria of a heart. The catheter is configured to transition to a geometry with a first shape setting mandrel and/or deflection geometry and a second shape setting mandrel and/or deflection geometry, where the first geometry is different than the second geometry. The catheter is used to ablate tissue in the right atrium using at least the first geometry and also ablate tissue in the left atrium using at least the second geometry.

**.** According to alternative embodiments, a catheter for performing a medical procedure on a patient is provided. The catheter, or catheter assembly or equipment 100, comprises an elongate shaft with a proximal portion including a proximal end and a distal end, and a distal portion with a proximal end and a distal end. The catheter further comprises a shape setting mandrel and/or deflection assembly configured to shape the distal portion in a first direction in a first geometry and a second direction in a second geometry, wherein the first and second geometries are different. The catheter further includes a functional element fixedly mounted to the distal portion.

**.** Therefore, it is the object of the present invention to provide an ablation equipment or assembly having structural and functional features such as to meet the aforementioned needs and overcome the drawbacks mentioned above with reference to the devices of the prior art.

**.** These and other objects are achieved by a device according to claim **1.**

**.** Some advantageous embodiments are the subject of the dependent claims.

### . Drawings

**.** Further features and advantages of the invention will become apparent from the description provided below of exemplary embodiment thereof, given by way of non-limiting example, with reference to the accompanying drawings, in which:

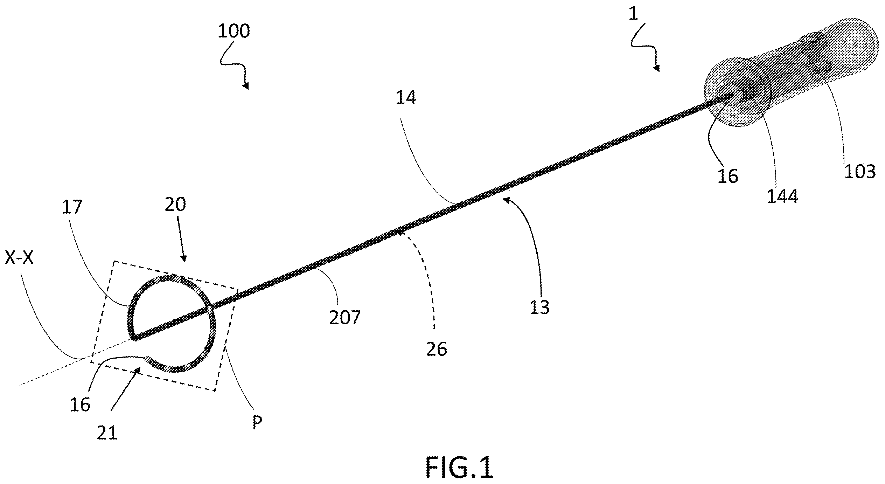

- **Figure 1** is a perspective view of an ablation assembly according to an embodiment of the present invention showing an ablation catheter having an elongate shaft, and a shape setting mandrel having disposed within the ablation catheter;

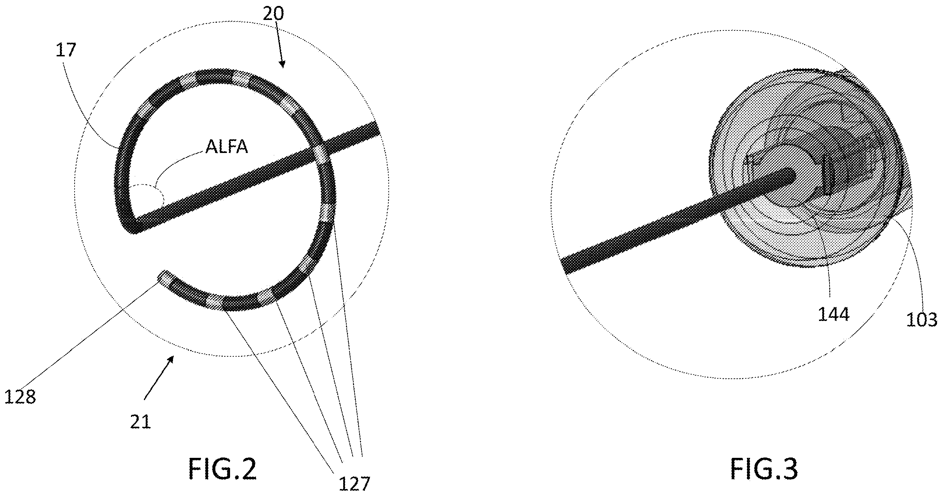

- **Figure 2** is a detail of the ablation assembly of figure 1 showing a shaft distal portion of the elongate shaft;
- **Figure 3** is a detail of the ablation assembly of figure 1 showing an handle and a steering device connected to the handle and to the elongate shaft;

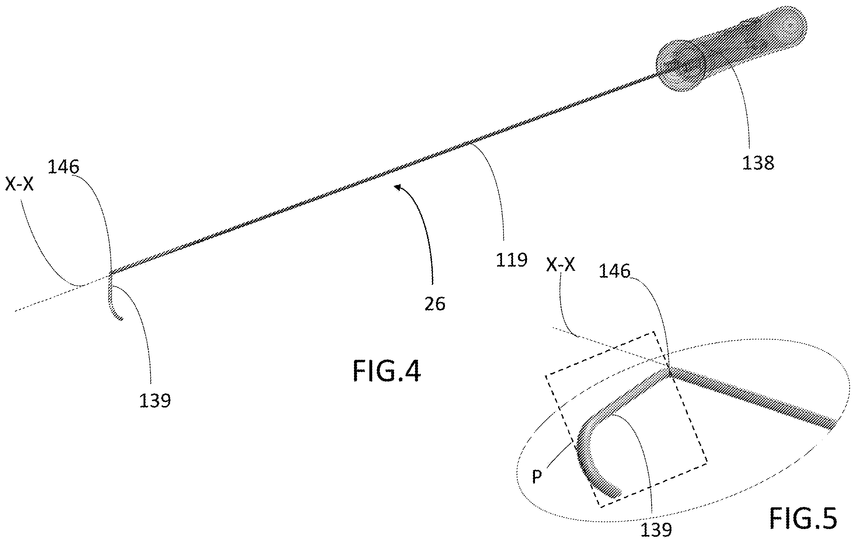

- **Figure 4** shows an ablation assembly according to the invention, wherein the elongate shaft and the steering device are omitted to show the shape setting mandrel partially inserted into the handle, wherein the shape setting mandrel has a bend preformed configuration;
- **Figure 5** is a detail of the shape setting mandrel of figure 4 showing a mandrel distal portion in the bend preformed configuration;

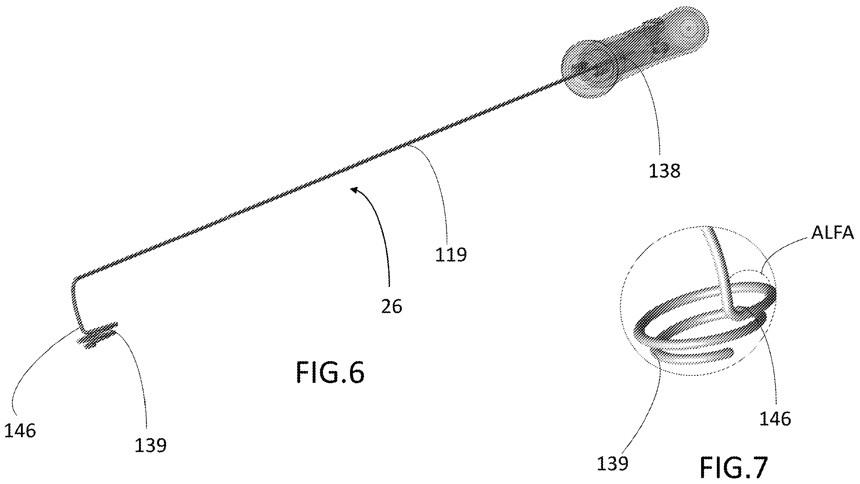

- **Figure 6** shows an ablation assembly according to the invention, wherein the elongate shaft and the steering device are omitted to show the shape setting mandrel partially inserted into the handle, wherein the shape setting mandrel has a spiral bend preformed configuration;
- **Figure 7** is a detail of the shape setting mandrel of figure 6 showing a mandrel distal portion in the spiral bend preformed configuration;

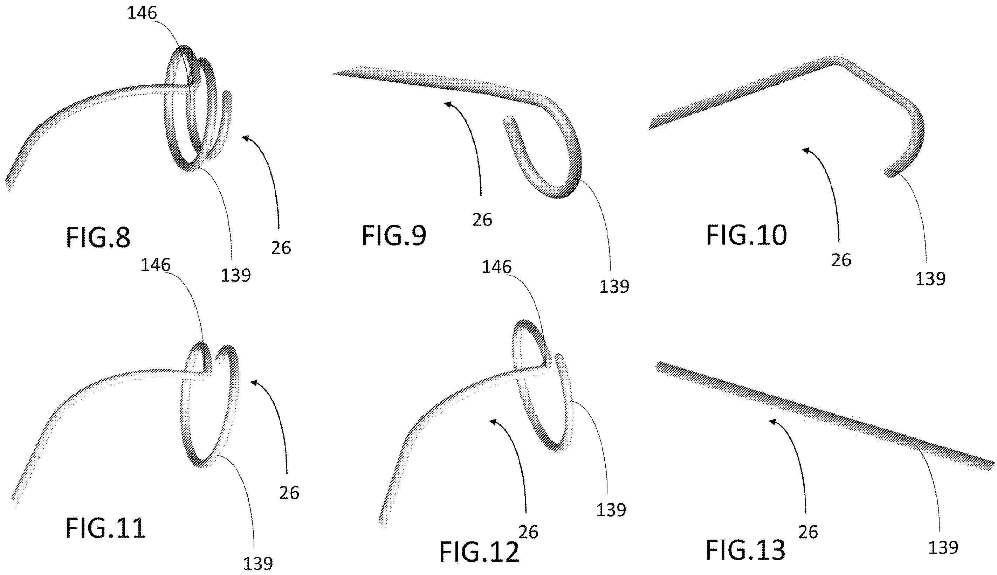

- **Figures 8-13** show different preformed configuration of a shape setting mandrel and the ablation assembly of the present invention;

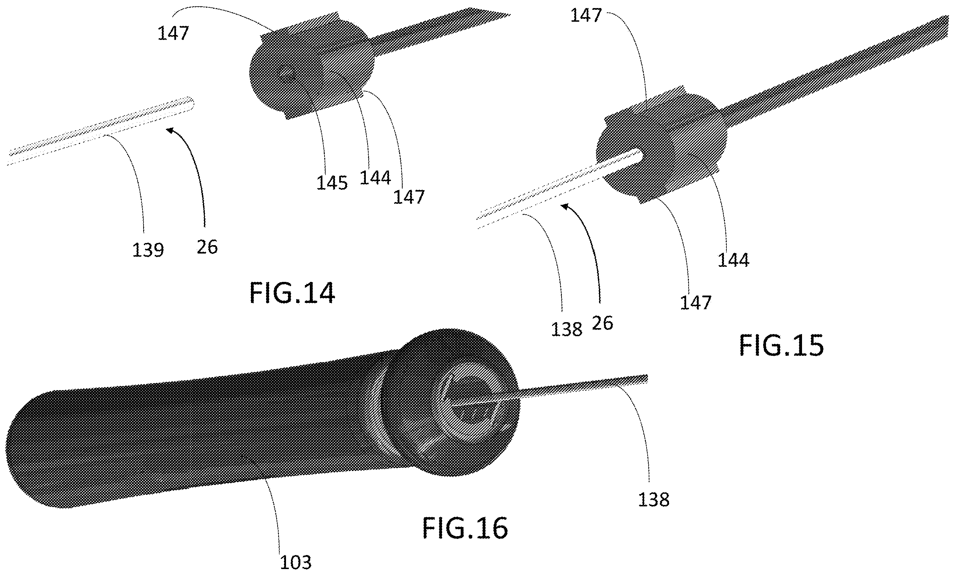

- **Figures 14-15** show a sequence of insertion of a shape setting mandrel in a loaded straight configuration within the elongate shaft of the ablation catheter of Figure 1, wherein the shape setting mandrel slides into a steering device connectable to an handle of the ablation catheter;
- **Figures 16** is a partial perspective view of the ablation assembly according to the invention, wherein the steering device and elongate shaft of figure 14 and 15 are omitted in order to show a proximal part of the mandrel disposed within the handle of the ablation catheter;

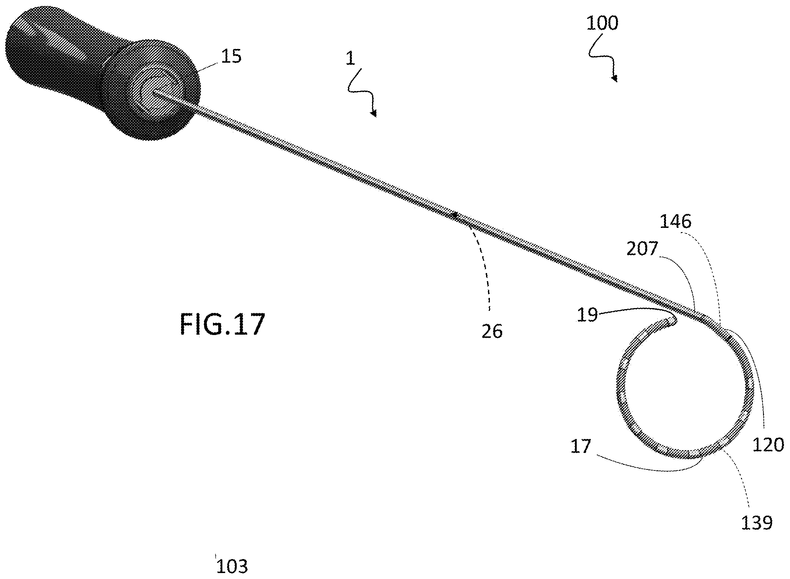

- **Figure 17** is a perspective view of an ablation assembly according to another embodiment of the present invention showing an ablation catheter having an elongate shaft, and a shape setting mandrel having a circular preformed configuration disposed within the ablation catheter;

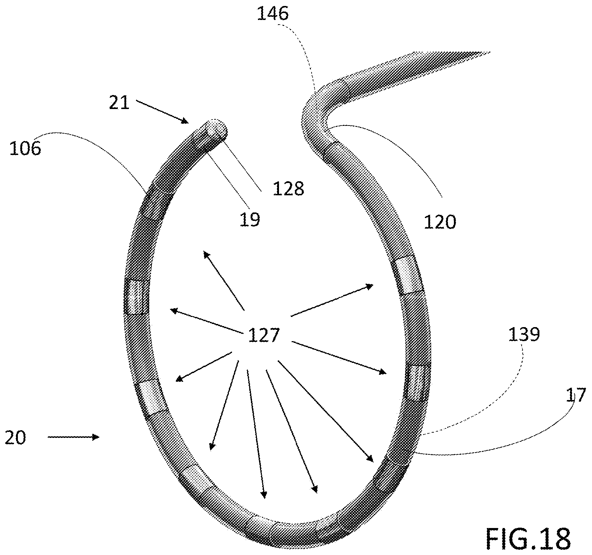

- **Figure 18** is a detail of the ablation assembly of figure 1 showing a shaft distal portion of the elongate shaft;

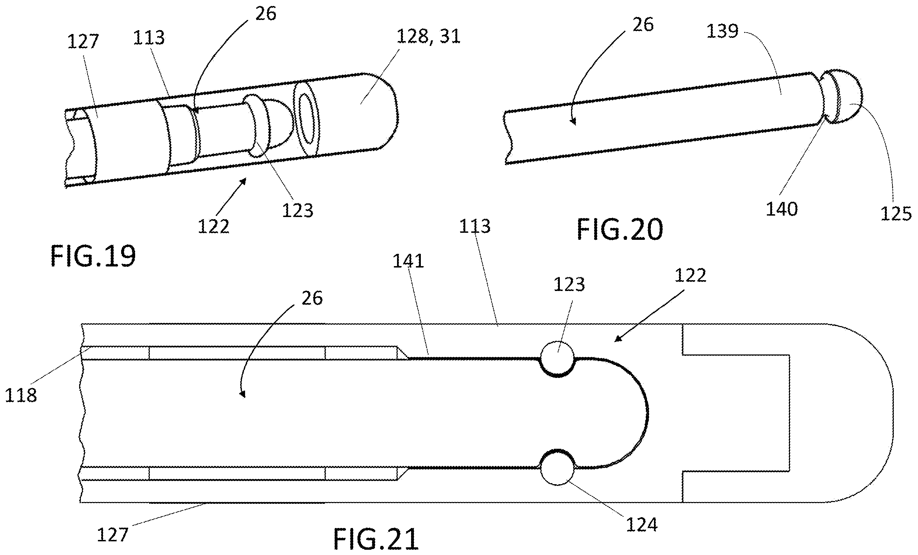

- **Figure 19** is perspective and schematic view of a shaft distal portion of the ablation catheter of the assembly according to the invention, that shows a locking mechanism between a shape setting mandrel and the shaft distal portion;
- **Figure 20** shows in detail the shape setting mandrel of figure 19 having a ball tip;
- **Figure 21** is a section view of the shaft distal portion of figure 19 along a longitudinal direction showing in detail the elements of the locking mechanism;

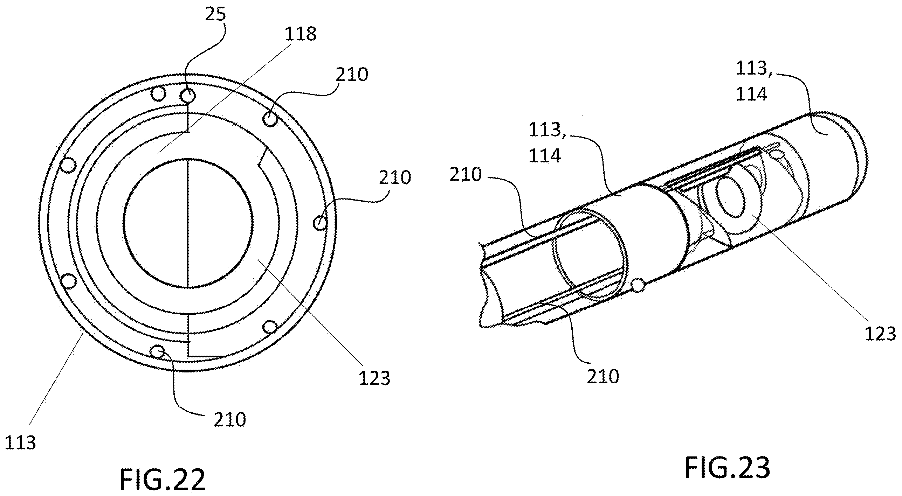

- **Figure 22** is a cross-sectional view of the shaft distal portion of figure 19, wherein the shape setting mandrel is omitted;
- **Figure 23** is a perspective view of the shaft distal portion of figure 19, wherein some external elements are partially removed and the shape setting mandrel is omitted to show the inner lumen of the catheter;

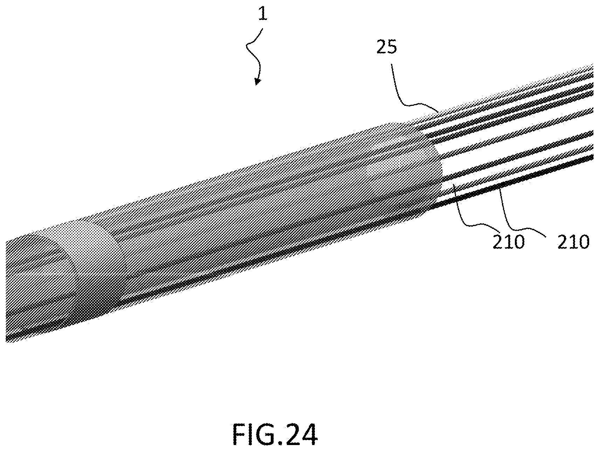

- **Figure 24** is a perspective schematic view of a portion of the ablation catheter wherein are shown electrical connectors disposed within the ablation catheter;
- **Figure 25** is a perspective view of a distal portion of an ablation assembly according to a further embodiment of the present invention showing an ablation catheter having an elongate shaft, and a shape setting mandrel having a circular preformed configuration disposed with its distal portion beyond a distal end of the elongate shaft;

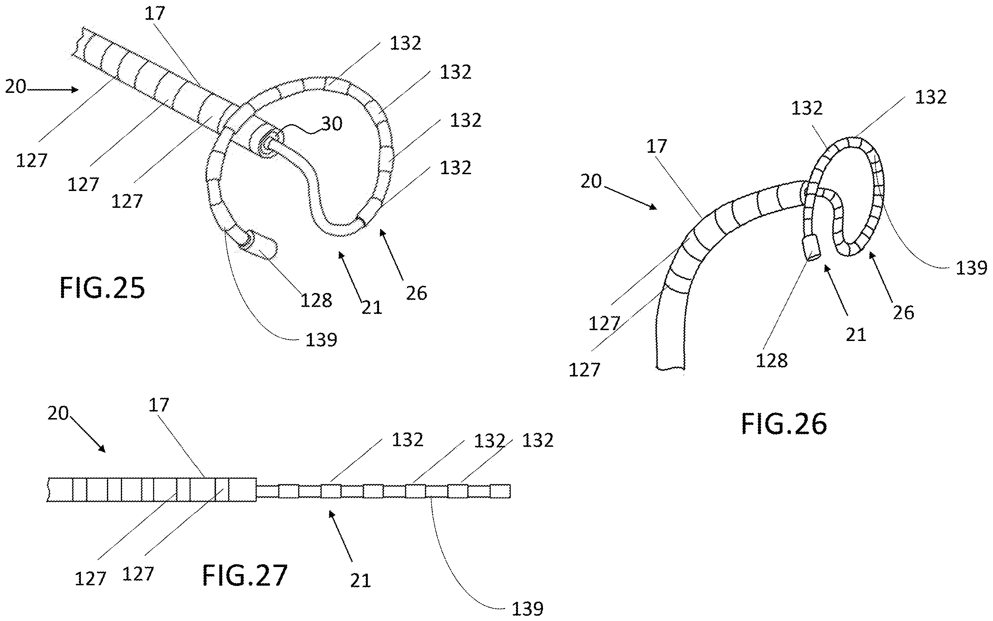

- **Figure 26** is a perspective view of a distal portion of an ablation assembly according to a further embodiment of the present invention showing an ablation catheter having an elongate shaft, and a shape setting mandrel having a circular preformed configuration disposed with its distal portion beyond a distal end of the elongate shaft, and wherein a distal portion of the elongate shaft is deflected in a deflection direction, wherein the shape setting mandrel comprises a plurality of mandrel electrodes disposed along its length, and the elongate shaft comprises a plurality of shaft electrodes;
- **Figure 27** is a side view of the ablation assembly of figure 25;

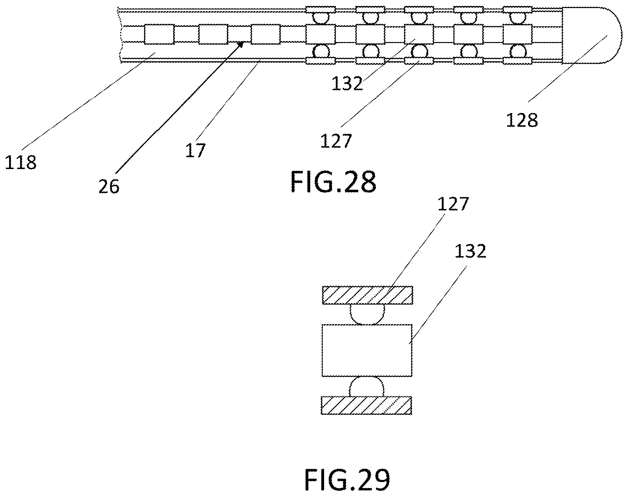

- **Figure 28** is a section view of the ablation assembly of figure 25, wherein the distal portion of the shape setting mandrel is fully inserted into the elongate shaft;
- **Figure 29** shows a detail of figure 28, showing an electrical connection between the mandrel electrodes and the shaft electrodes;

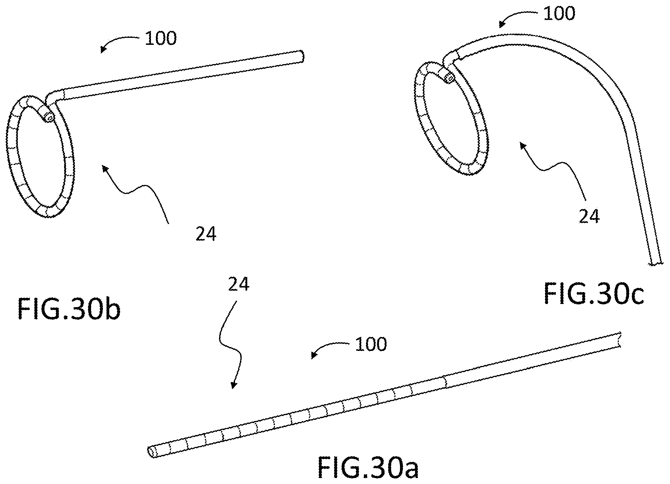

- **Figure 30a-30c** shows a shape setting mandrel respectively in a loaded straight configuration, in a preformed circular configuration, and in a preformed circular and bent configuration;

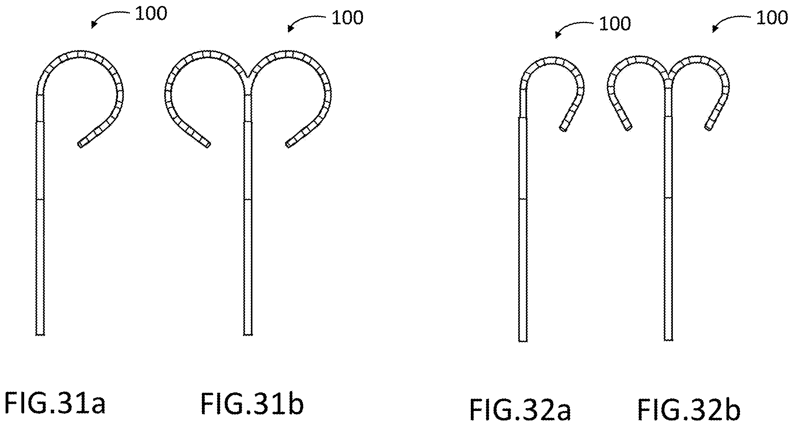

- **Figures 31a-31b and 32a-32b** show a plurality of shape setting mandrels having different preformed configurations;

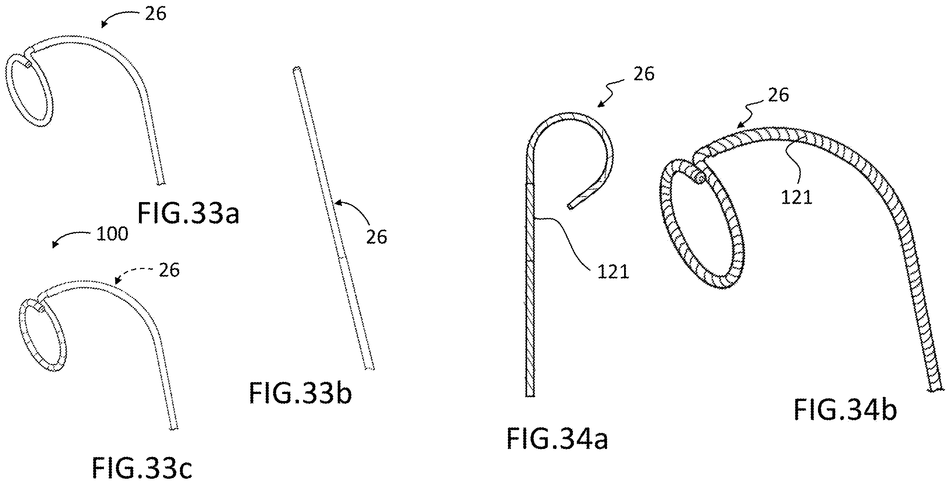

- **Figure 33a-33c** shows a shape setting mandrel respectively in a preformed circular and bent configuration and in a loaded straight configuration, and the shape setting mandrel in the preformed circular and bent configuration disposed within an ablation catheter;
- **Figure 34a-34b** shows two shape setting mandrels coupled to a respective heating element, wherein the heating element is configured to apply heat to the shape setting mandrel to modify shape of the shape setting mandrel from a loaded configuration to a preformed configuration;

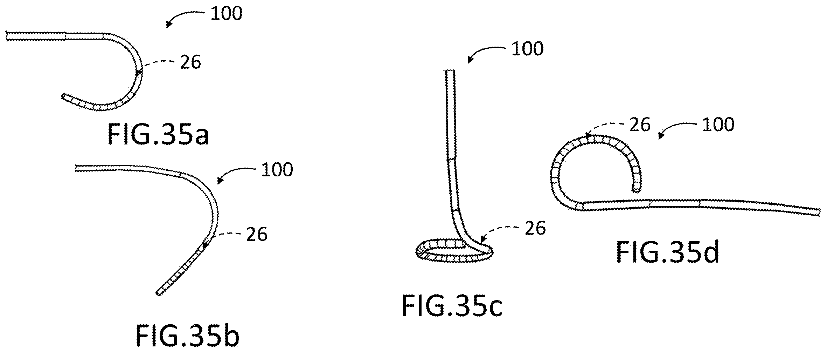

- **Figure 35a-35d** show different curves and 2-D and 3-D configurations of a distal portion of an ablation catheter with a shape setting mandrel disposed within the distal portion of the ablation catheter;

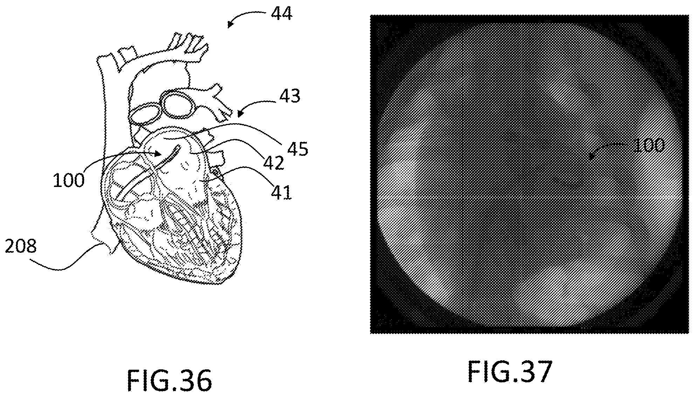

- **Figure 36** shows an ablation assembly according to the present invention disposed within an heart, wherein a shape setting mandrel is fully inserted in a distal portion of the ablation catheter shaft;
- **Figure 37** shows a radiography of an ablation assembly according to the present invention, wherein a catheter distal portion is shape set as a pre-formed configuration of a shape setting catheter fully inserted into the catheter distal portion;

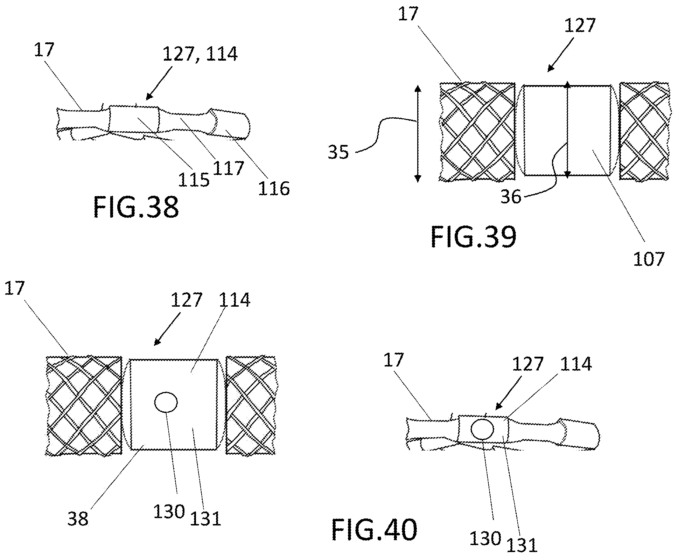

- **Figure 38** shows a plurality of shaft electrodes fixedly disposed and spaced apart along a catheter shaft distal portion according to an embodiment, wherein said shaft electrodes are biased in circular configuration on the catheter shaft;
- **Figure 39** shows a shaft electrode disposed along the catheter shaft wherein the shaft electrode catheter is tubular and forms a part of the catheter shaft;
- **Figure 40** shows the shaft electrodes of figure 38 an figure 39 in a bipolar configuration;

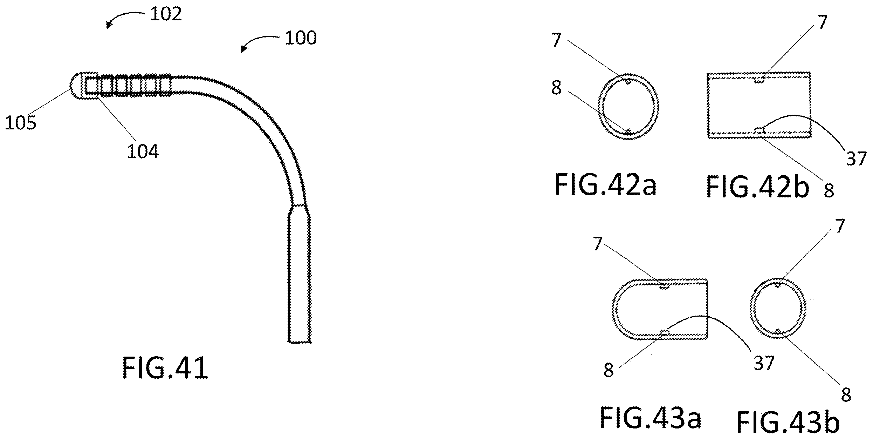

- **Figure 41** is a side view of a distal portion of an ablation catheter according to the invention comprising a plurality of shaft electrodes and an tip electrode;
- **Figure 42a-42b** shows a cross-section view and a longitudinal section view of the ablation catheter of figure 41, showing the electrical connections for electrical wires for connecting one of the shaft electrodes to a single power source;
- **Figure 43a-43b** shows a cross-section view and a longitudinal section view of the ablation catheter of figure 41, showing the electrical connections for electrical wires for connecting the tip electrode to a single power source;

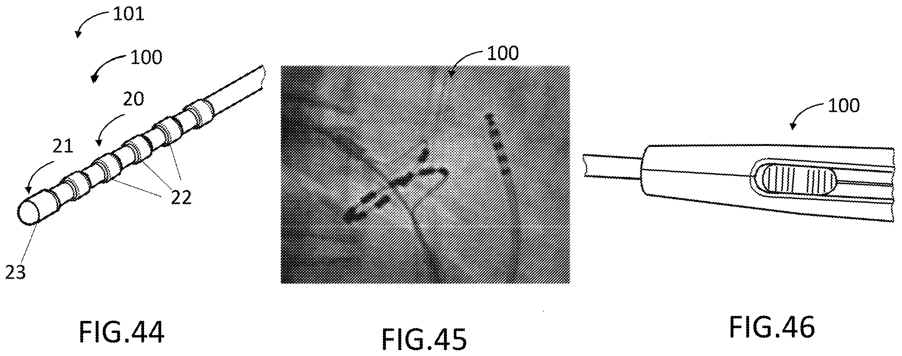

- **Figure 44** is a perspective view of a shaft distal portion of an ablation catheter according to the invention comprising a plurality of shaft electrodes and a tip electrode, wherein the outer profile or diameter of the shaft electrodes and the outer profile of the tip electrode are bigger than the outer profile or diameter of the shaft distal portion;
- **Figure 45** shows a radiography of an ablation assembly according to the present invention, wherein a catheter distal portion is shown in two different shapes and deflections;
- **Figure 46** shows a side view of an ablation catheter handle of the ablation assembly according to an embodiment;

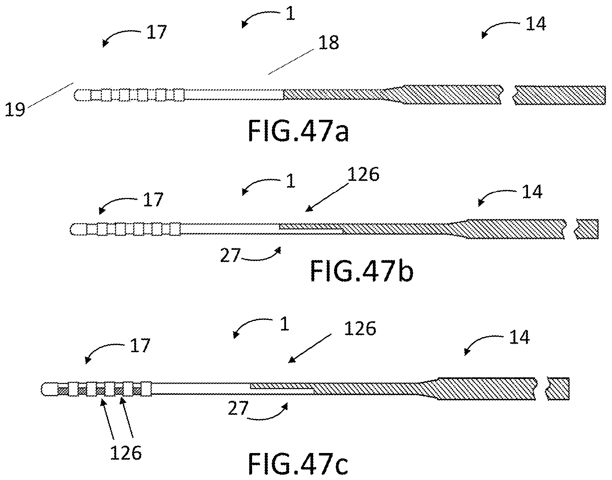

- **Figure 47a-47c** shows a schematic lateral view of three different configuration of an ablation catheter, wherein the ablation catheter have different stiffness along its length, wherein the ablation catheter is symmetrical deflectable, or asymmetrical deflectable, and/or wherein the plurality of catheter shaft portions between two electrodes have a first stiffness, the remaining portion of the shaft distal portion have a second stiffness and the shaft proximal portion have a third stiffness;

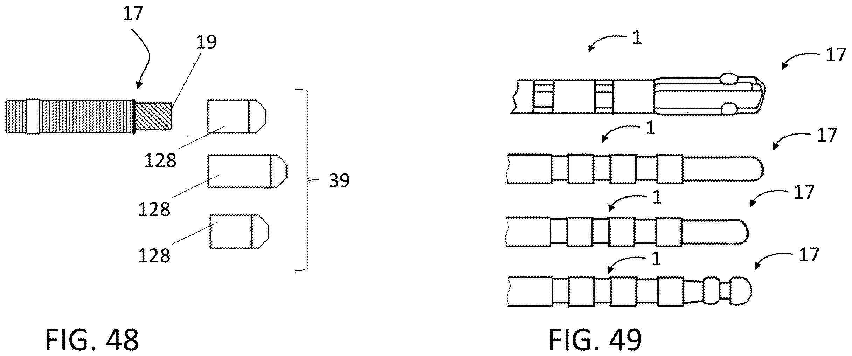

- **Figure 48** shows a side view of a shaft distal portion and a set of different tip electrodes, wherein each tip electrode can be coupled to the shaft distal portion;
- **Figure 49** shows a side view of different shaft distal portion of different ablation catheters;

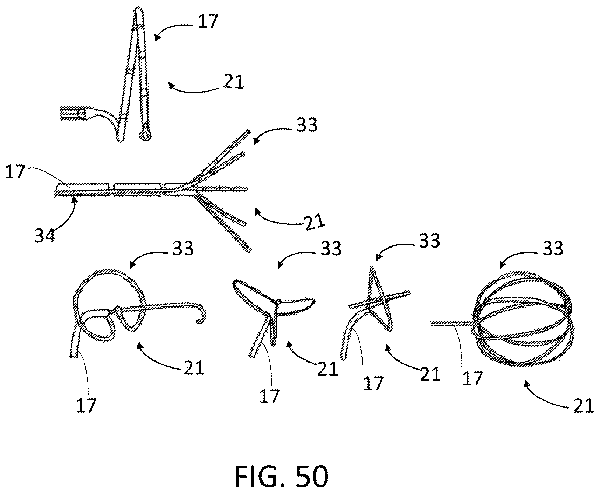

- **Figure 50** shows a perspective view of different distal ablation assemblies which can be coupled to the shaft distal portion;

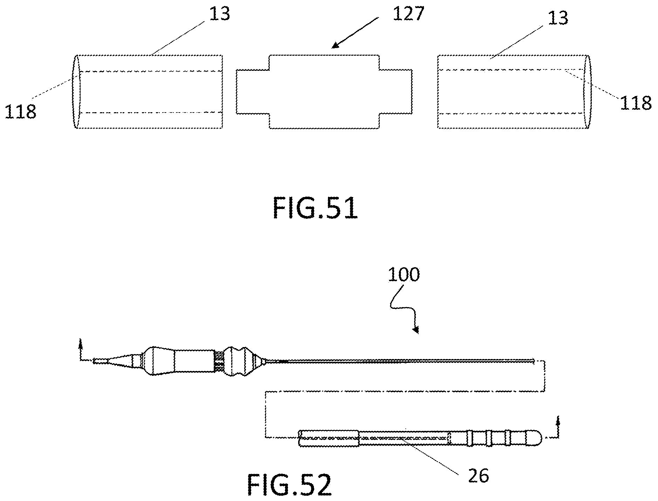

- **Figure 51** shows an exploded side view of a tubular shaft electrode and two portions of a shaft distal portion;
- **Figure 52** shows a side schematic view of an ablation catheter assembly according to an embodiment;

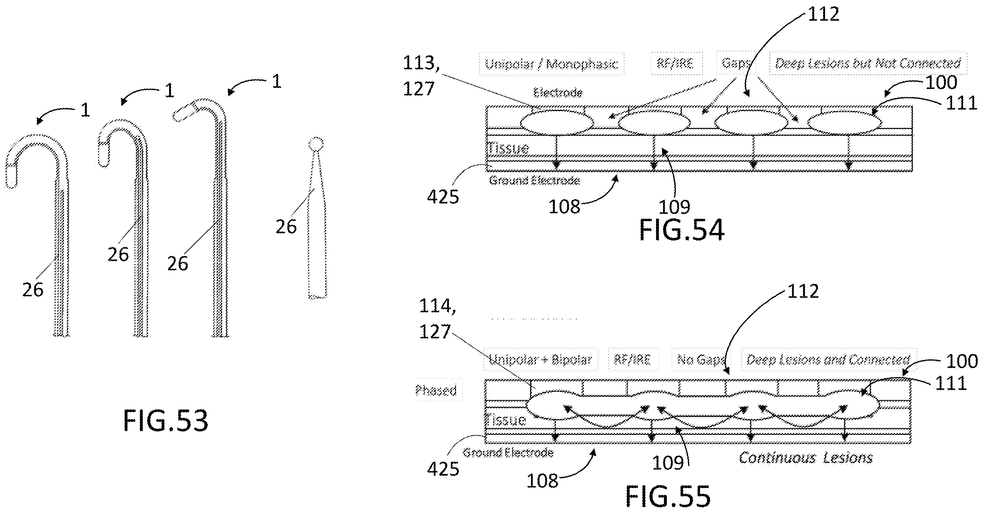

- **Figure 53** shows a section side view of different ablation catheters and different shape setting mandrels disposed within the ablation catheter, and a shape setting mandrel having a rounded distal end;
   **Figure 54** shows an example of operation of the ablation equipment of the invention to generate monopolar electric filed from each electrode with a ground electrode;
- **Figure 55** shows an example of operation of the ablation equipment of the invention to generate both a monopolar electric filed from each electrode with a ground electrode and a bipolar electric field between two contiguous electrodes;

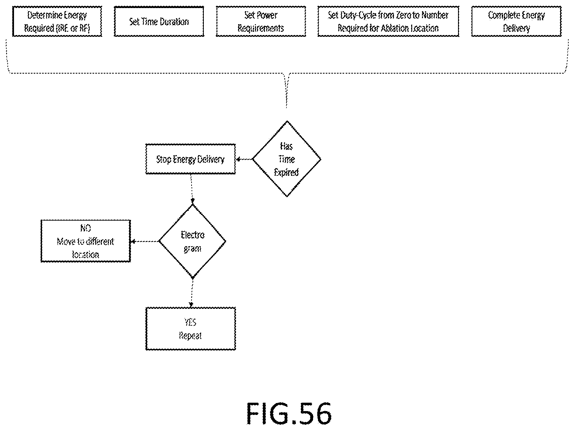

- **Figure 56** shows a flux diagram of a method for ablation with an ablation assembly of the present invention;

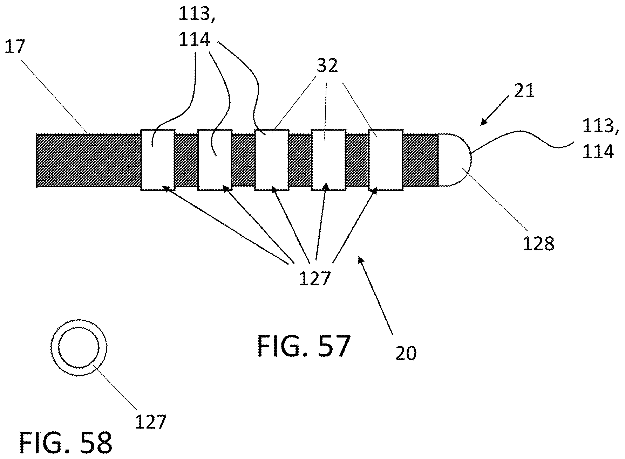

- **Figure 57** and 58 show a side view and a cross-sectional view, respectively, of the shaft distal portion of a catheter showing a shaft ablation assembly comprising a plurality of electrodes according to a first embodiment;

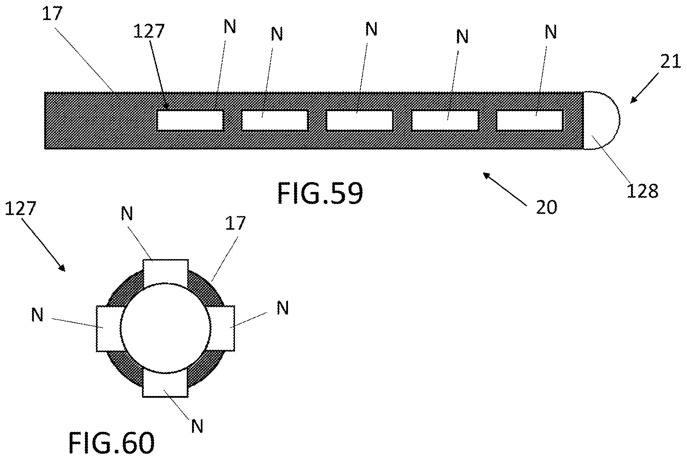

- **Figure 59 and 60** show a side view and a cross-sectional view, respectively, of the shaft distal portion of a catheter, showing a shaft ablation assembly comprising a plurality of electrodes according to a second embodiment;

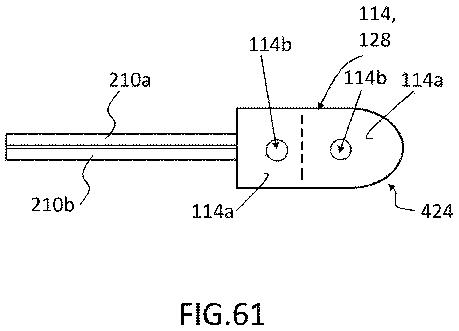

- **Figure 61** shows an embodiment of a bipolar electrode comprising a first electrode having an electrode body that delimits an internal compartment of the first electrode accessible from the outside and a second point like electrode housed in said internal compartment of the first electrode;

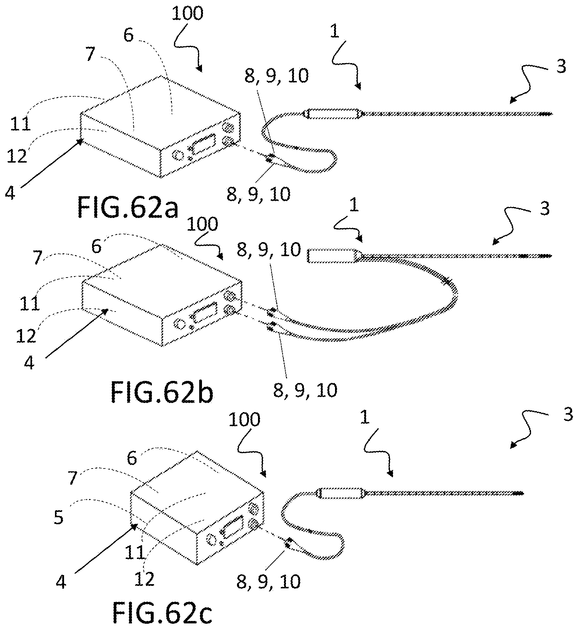

- **Figures 62A, 62B, 62C** shows an ablation equipment comprising a single power source, a single control unit and a power unit, an ablation catheter and a shape setting mandrel disposed in the ablation catheter, wherein are shown in three different electrical connection configurations between the ablation catheter and the single power source;

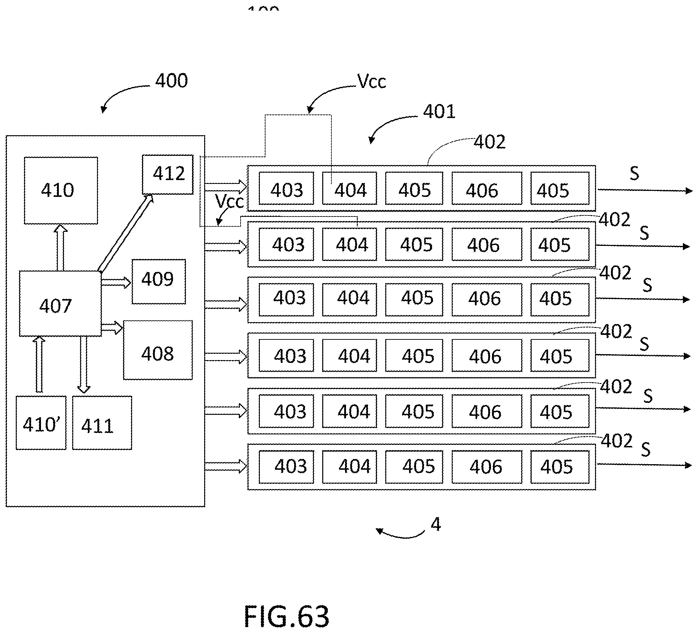

- **Figure 63** shows a block diagram of a single power source of an ablation equipment comprising a single control unit and a power unit;

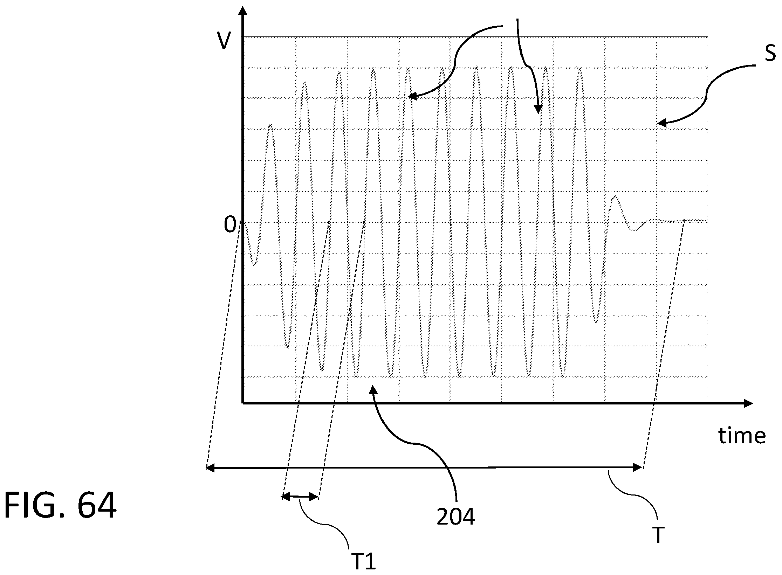

- **Figure 64** shows an example of a sinewave electrical signal generated by the single power source of figure 63;

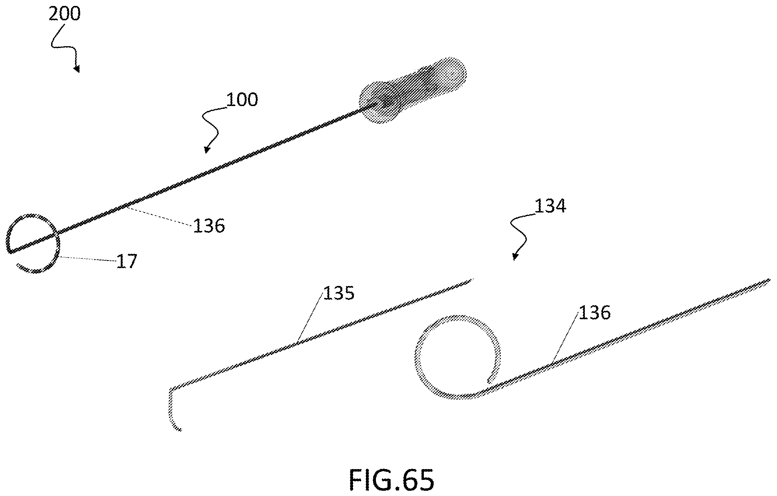

- **Figure 65** shows an ablation kit comprising at least an ablation assembly and a set of shape setting mandrels;

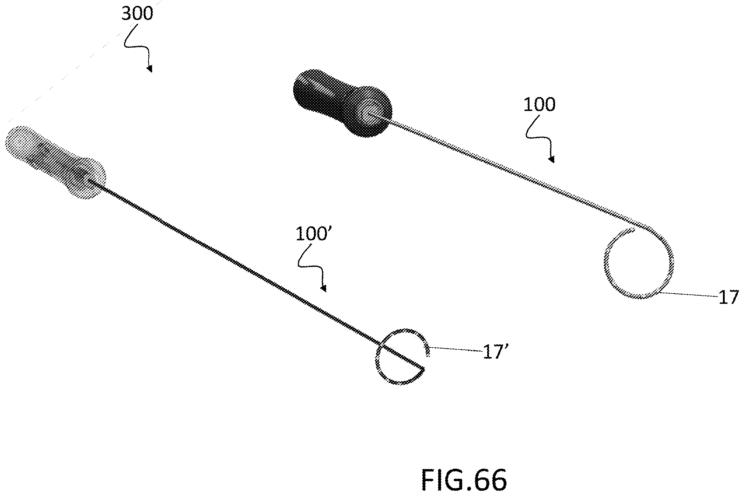

- **Figure 66** shows an ablation catheter kit comprising a first ablation assembly and a second ablation assembly having different deflection configurations

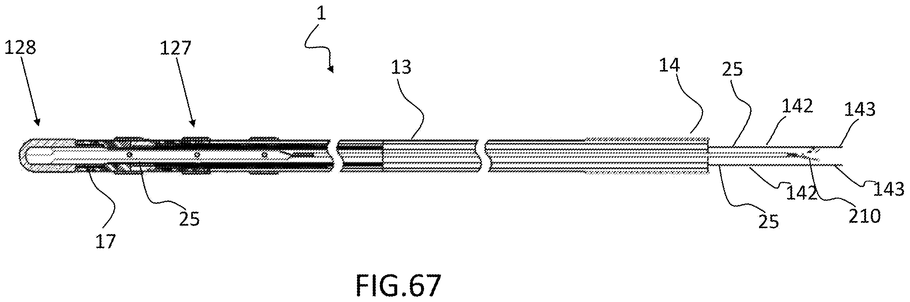

- **Figure 67** shows an ablation catheter in a schematic section view along its length, wherein steering wires and electrical conductors wires are shown.

### . Description of some preferred embodiments

**.** The present invention can be understood more readily by reference to the following detailed description, examples, drawing, and their previous and following description. However, before the present devices, systems, and/or methods are disclosed and described, it is to be understood that this invention is not limited to the specific devices, systems, and/or methods disclosed unless otherwise specified, as such can, of course, vary, as long as they fall within the scope of the appended claims. It is also to be understood that the terminology used herein is for the purpose of describing particular aspects only and is not intended to be limiting.

**.** In accordance with a general embodiment, an ablation equipment 100 to treat target regions of tissue 41 in organs 44, comprises an ablation catheter 1 and a single power source 4.

**.** Said ablation catheter 1 comprises a catheter elongated shaft 13 comprising at least an elongated shaft distal portion 17.

**.** Said catheter elongated shaft 13 comprises a flexible body 207 to navigate through body vessels 208.

**.** Said ablation catheter 1 further comprises a shaft ablation assembly 20 disposed at said elongated shaft distal portion 17.

**.** Said shaft ablation assembly 20 comprises at least a plurality of electrodes 127, 113 or 114 fixedly disposed at said elongated shaft distal portion 17.

**.** All electrodes of said at least a plurality 127, 113 or 114 are electrically powered by said single power source 4 through an electric signal S to deliver both non-thermal energy for treating the tissue 41 and thermal energy for ablating the tissue 41.

**.** Said electric signal S comprises a sinusoidal wave. According to an embodiment, the electric signal S comprises a plurality of sinusoidal waves. In a further embodiment, the electric signal is a voltage signal.

**.** Said single power source 4, when requested, changes continuously said electric signal S in order to power the said least a plurality of electrodes 127, 113 or 114 to deliver from a non-thermal energy to a thermal energy, and vice versa, or to deliver at the same time a combination of thermal energy and non-thermal energy.

**.** In accordance with an alternative embodiment, said single power source 4 comprises a single control unit 400 and a power unit 401 for generating said electric signal S comprising a sinusoidal wave.

**.** Said power unit 401 is electrically connected to all electrodes of said at least a plurality of electrodes 127, 113 or 114.

**.** In accordance with an alternative embodiment, said power unit 401 being electrically connected to all electrodes of said at least a plurality of electrodes 127, 113 or 114.

**.** In accordance with an alternative embodiment, said electric signal S is supplied to the electrodes of said plurality 127, 113 or 114 during a time interval T.

**.** In accordance with an alternative embodiment, said electric signal S is a sinusoidal pulse train 204 comprising two or more basic sine waves BSW in said time interval T.

**.** In accordance with an alternative embodiment, each basic sine wave BSW consisting in one positive half-wave and one negative half-wave.

**.** In accordance with an alternative embodiment, each basic sine wave BSW having a duration equal to a first time interval T1

**.** In accordance with an alternative embodiment, said single control unit 400 is configured to drive the power unit 401 to modify the duration of the first time interval T1 of the basic sine wave BSW to change the electric energy level associated to the electric signal S.

**.** In accordance with an alternative embodiment, said first time interval T1 is selected in the range of 1 µsec - 80.000msec, particularly in the range of 75µsec - 20.000msec.

**.** In accordance with an alternative embodiment, said first time interval T1 is selected in the range of 20µsec - 100µsec.

**.** In accordance with an alternative embodiment, the sinusoidal pulse train electric signal S is supplied to the electrodes 127, 113 or 114 during a time interval T selected in the range of 100µsec - 100sec.

**.** In accordance with an alternative embodiment, said single control unit 400 is configured to drive the power unit 401 to modify the number of pulses in the sinusoidal pulse train 204 to change the electric energy level associated to the electric signal S.

**.** In accordance with an alternative embodiment, said sinusoidal pulse train electric signal S comprises from two to twenty-five basic sine waves BSW in said time interval T.

**.** In accordance with an alternative embodiment, said electric signal S comprising a sinusoidal wave is a voltage signal, a peak-to-peak mean amplitude of each basic sine wave BSW is in the range of 1.000 V to 2.000 V.

**.** In accordance with an alternative embodiment, the electrodes of said at least a plurality 127, 113 or 114 are electrically powered by said single power source 4 to deliver a voltage to treat the target regions of tissue 41 which is selected in the range of 100 V/cm - 7000 V/cm, particularly selected in the range of 200 V/cm - 2000 V/cm or selected in the range of 300 V/cm - 1000 V/cm.

**.** In accordance with an alternative embodiment, said power unit 401 comprises a power module 402. Said power module 402 comprises:
**.** a drive circuit block 403 controlled by the single control unit 400 for generating said electric signal S starting from a supply voltage signal Vcc provided by the single control unit 400;
**.** a selecting block 404 selectively controlled by said drive circuit block 403 to change continuously the electric energy level associated to said signal S;
**.** a filtering and electrical isolation block 405, 406.

**.** In accordance with an alternative embodiment, said single control unit 400 comprises a Microprocessor 407 configured to control a variable High Voltage Power Supply block 408 and a Programmable Logic Controller block 409.

**.** Said variable High Voltage Power Supply block 408 being configured to provide said supply voltage signal Vcc to the power module 402 for generating said electric signal S.

**.** Said Programmable Logic Controller block 409 being configured to generate drive signals to control the drive circuit block 403 of the power module 402.

**.** In accordance with an alternative embodiment, said single control unit 400 further comprises:
**.** a Video interface and Push Button block 410, 410' controlled by the Microprocessor 407 to set parameters of the equipment 100 and display the selected parameters;
**.** a Watch Dog block 411 for controlling proper functioning of the Microprocessor 407;
**.** an Audio interface block 412 for providing audio information representative of correctness of the ablation process and/or errors occurred.

**.** In accordance with an alternative embodiment, said power unit 401 comprises one or more power modules 402 equal to each other.

**.** In accordance with an alternative embodiment, at least one of said electrodes 127, 113 is a monopolar electrode 113, and said monopolar electrode 113 of said at least a plurality of electrodes is electrically connected to only one power module 402 of said power unit 401.

**.** In accordance with an alternative embodiment, at least two of said electrodes 127, 114 are electrically connected to form a bipolar electrodes 114, and said bipolar electrodes 114 of said at least a plurality of electrodes are electrically connected separately to respective power module 402 selectable among the power modules of said power unit 401.

**.** In accordance with an alternative embodiment, said single control unit 400 is configured to drive the power unit 401 to modify the number of pulses in the pulse train 204 to change the electric energy level associated to the sinusoidal signal S.

**.** In accordance with an alternative embodiment, each monopolar electrode 113 of said least a plurality of electrodes is electrically connected to the corresponding power module 402 of said power unit 401 by a single wire 210 welded to the monopolar electrode 113.

**.** In accordance with an alternative embodiment, each bipolar electrode 114 of said least a plurality of electrodes is electrically connected to the two selected power modules 402 of said power unit 401 by two wires 210 welded to the bipolar electrode 114.

**.** In accordance with an alternative embodiment, at least one electrode of said least a plurality of electrodes 127 comprises two conductive portions N electrically isolated from each other.

**.** In accordance with an alternative embodiment, at least one electrode of said least a plurality of electrodes 127 comprises four conductive portions N electrically isolated from each other.

**.** In accordance with an alternative embodiment, the non-thermal energy is irreversible electroporation energy or IRE, the thermal energy is radiofrequency energy or RF.

**.** In accordance with an alternative embodiment, said single power source 4 is powered by a battery or is connected to a standard wall outlet of an AC electrical power grid capable of producing 110 volts or 240 volts.

**.** In accordance with an alternative embodiment, said least two electrodes 127, 114 electrically connected to form a bipolar electrodes 114 comprise:
**.** a first electrode 114a connected to a first power module 402 of said power unit 401 by a first wire 210a, said first electrode 114a having an electrode body 424 that delimits an internal compartment of the first electrode 114a accessible from the outside of the first electrode 114a;
**.** a second point like electrode 114b connected to a second power module 402 of said power unit 401 by a second wire 210b, said second point like electrode 114b being housed in said internal compartment of the first electrode 114a.

**.** In accordance with an alternative embodiment, said ablation catheter 1 comprises an elongate shaft 13 having a longitudinal main direction X-X. Said elongate shaft 13 comprises at least shaft distal portion 17. Said shaft distal portion 17 comprises a shaft distal portion distal end 19.

**.** Said ablation catheter 1 comprises an inner lumen 118 arranged within the elongate shaft 13.

**.** Said ablation catheter 1 comprises a shaft ablation assembly 20 fixedly disposed at said shaft distal portion 17, the shaft ablation assembly 20 being configured to deliver both thermal energy for ablating said tissue 41 and non-thermal energy for treating said tissue 41.

**.** Said equipment 100 comprises at least a shape setting mandrel 26 is disposed within the ablation catheter 1. The shape setting mandrel 26 is insertable within the inner lumen 118 and removable from the inner lumen 118,

**.** The shape setting mandrel 26 is free to move in respect of the inner lumen 118 avoiding any constraint with said shaft distal portion 17 during the shape setting mandrel insertion.

**.** The shape setting mandrel 26 comprises at least a pre-shaped configuration and the shape setting mandrel 26 is reversibly deformable between at least a straight loaded configuration and said pre-shaped configuration.

**.** When the shape setting mandrel 26 is fully inserted in the shaft distal portion 17, the shape setting mandrel 26 is configured to shape set said shaft distal portion 17 with said pre-shaped configuration.

**.** In accordance with an alternative embodiment, said shaft distal portion 17 is elastically deformable.

**.** In accordance with an alternative embodiment, when the shape setting mandrel 26 is fully inserted in the shaft distal portion 17, said shaft distal portion 17 is configured to conform to said pre-shaped configuration.

**.** In accordance with an alternative embodiment, when the shape setting mandrel 26 is fully inserted in the shaft distal portion 17 it is defined a mandrel fully inserted position.

**.** While the shape setting mandrel 26 slides within the inner lumen 118 towards said mandrel fully inserted position, the shape setting mandrel 26 is configured to variably shape set the shaft distal portion 17 passing from said loaded straight configuration to said pre-shaped configuration.

**.** In accordance with an alternative embodiment, when the shape setting mandrel 26 is fully inserted in the shaft distal portion 17, said shape setting mandrel 26 deform said shaft distal portion 17 at least in a shaft distal portion plane P.

**.** In accordance with an alternative embodiment, said ablation catheter 1 comprises a catheter bend portion 120 proximal to the shaft ablation assembly 20, wherein said catheter bend portion 120 is configured to realize an elbow that steer said shaft distal portion plane P with respect to said longitudinal main direction X-X.

**.** In accordance with an alternative embodiment, at least when the shape setting mandrel 26 is fully inserted in the shaft distal portion 17 said shaft distal portion 17 forms an acute angle ALFA with respect to the shaft longitudinal main direction X-X.

**.** In accordance with an alternative embodiment, wherein when the shape setting mandrel 26 is fully inserted in the shaft distal portion 17, the shape setting mandrel 26 is configured to bend at said catheter bend portion 120.

**.** In accordance with an alternative embodiment, said shape setting mandrel 26 in said pre-shaped configuration comprises a mandrel bend portion 146, and when said shape setting mandrel 26 is fully inserted in said shaft distal portion 17, said mandrel bend portion 146 is disposed in correspondence of said catheter bend portion 120 performing said catheter bend portion 120.

**.** In accordance with an alternative embodiment, when the shape setting mandrel 26 is fully inserted in the shaft distal portion 17, the shaft distal portion 17 takes a circular configuration.

**.** In accordance with an alternative embodiment, the shape setting mandrel 26 comprises a mandrel elastic body 119 capable to deform into at least said straight loaded configuration and to return to said pre-shaped configuration.

**.** In accordance with an alternative embodiment, the shape setting mandrel 26 is made of at least a shape memory alloy.

**.** In accordance with an alternative embodiment, said assembly 100 comprises a mandrel heating element 121 coupled to said shape setting mandrel 26, wherein said heating element 121 is configured to apply heat to said shape setting mandrel 26 so that shape setting mandrel 26 changes shape configuration from said loaded straight configuration to said pre-shaped configuration.

**.** In accordance with an alternative embodiment, said ablation assembly 100 comprises a locking mechanism 122 configured to lock said shape setting mandrel 26 to said shaft distal portion 17 when said shape setting mandrel 26 is in said mandrel fully inserted position.

**.** In accordance with an alternative embodiment, said locking mechanism 122 comprises a retention element 123 that reversibly locks said shape setting mandrel 26 in said mandrel fully inserted position.

**.** In accordance with an alternative embodiment, said retention element 123 is configured to release said shape setting mandrel 26 from said mandrel fully inserted position when a pull force is applied to said shape setting mandrel 26.

**.** In accordance with an alternative embodiment, said retention element 123 is made of metal, metal alloy, rubber or polymer.

**.** In accordance with an alternative embodiment, said shape setting mandrel 26 comprises a ball-tip 125 configured to engage said retention element 123 when said shape setting mandrel 26 is in said fully inserted position.

**.** In accordance with an alternative embodiment, said shape setting mandrel 26 comprises a mandrel distal portion 139.

**.** In accordance with an alternative embodiment, said mandrel distal portion 139 comprises a mandrel seat 140, wherein said retention element 123 is fixed to said shape setting mandrel 26 and partially housed in said mandrel seat 140.

**.** In accordance with an alternative embodiment, said inner lumen 118 proximal to said shaft distal portion distal end 19 presents a neck portion 141, wherein said retention element 123 interferes with said neck portion 141 to lock said shape setting mandrel 26 in said mandrel fully inserted position.

**.** In accordance with an alternative embodiment, said retention element 123 is an O-ring, wherein said mandrel seat 140 is toroidal.

**.** In accordance with an alternative embodiment, the shaft distal portion 17 is deflectable in one or more directions, in one or more deflections shapes and geometries.

**.** In accordance with an alternative embodiment, the shape setting mandrel 26 in the pre-shaped configuration is configured to maintain the deflections of the shaft distal portion 17 in a single plane.

**.** In accordance with an alternative embodiment, the deflection directions are symmetric deflection geometries or asymmetric deflection geometries.

**.** In accordance with an alternative embodiment, the elongate shaft 13 has difference in the stiffness of the shaft along its length.

**.** In accordance with an alternative embodiment, the elongate shaft 13 comprises a shaft proximal portion 14.

**.** In accordance with an alternative embodiment, said shaft proximal portion 14 is more rigid than said shaft distal portion 17.

**.** In accordance with an alternative embodiment, the elongate shaft 13 comprises a shaft transition portion 126 disposed between said shaft proximal portion 14 and said shaft distal portion 17.

**.** In accordance with an alternative embodiment, said shaft transition portion 126 is more rigid than said shaft distal portion 17 and less rigid then said shaft proximal portion 14.

**.** In accordance with an alternative embodiment, said elongate shaft 13 comprises shaft portions having different stiffness, wherein said elongate shaft 13 comprises at least one circumferentially dissymmetric stiffness portions between two of said shaft portions having different stiffness.

**.** In accordance with an alternative embodiment, said elongate shaft 13 is made of Pebax^{®}, or said elongate shaft 13 is braided and made of stainless steel flat wire brake and/or Nylon^{®} strand braid.

**.** In accordance with an alternative embodiment, said ablation catheter 1 comprises at least one steering wire 25 configured to deflect the shaft distal portion 17 in one or more deflection directions, wherein said at least one steering wire 25 is fixedly connected to said shaft distal portion 17.

**.** In accordance with an alternative embodiment, said at least one steering wire 25 comprises a wire proximal extension 142 that is arranged outside with respect to a shaft proximal portion 14.

**.** In accordance with an alternative embodiment, said wire proximal extension 142 comprises a wire gripping portion 143 configured to pull at least one the steering wire 25 for steering the shaft distal portion 17 with shape setting mandrel 26 fully inserted into the shaft distal portion 17.

**.** In accordance with an alternative embodiment, said shaft distal portion 17 comprises a shaft distal portion proximal end 18.

**.** In accordance with an alternative embodiment, said ablation catheter 1 comprises at least two steering wires 25.

**.** In accordance with an alternative embodiment, a first steering wire of said at least two steering wires 25 is fixedly connected proximal to the shaft distal portion distal end 19 or the shaft distal portion proximal end 18.

**.** In accordance with an alternative embodiment, a second steering wire of said at least two steering wires 25 is fixedly connected proximal to the shaft distal portion proximal end 18 or to the shaft distal portion distal end 19.

**.** In accordance with an alternative embodiment, a third steering wire of said at least two steering wires 25 is fixedly connected proximal to the shaft distal portion distal end 19 or to the shaft distal portion proximal end 18.

**.** In accordance with an alternative embodiment, a fourth steering wire of said at least two steering wires 25 is fixedly connected proximal to the shaft distal portion distal end 19 or to the shaft distal portion proximal end 18.

**.** In accordance with an alternative embodiment, said shape setting mandrel 26 comprises a mandrel proximal portion 138, wherein said mandrel proximal portion 138 is disposed outside said inner lumen 118 so that said shape setting mandrel 26 is drivable by a user.

**.** In accordance with an alternative embodiment, said elongate shaft 13 comprises a shaft proximal end 15.

**.** In accordance with an alternative embodiment, said ablation catheter 1 comprises a steering device 144 attached to said shaft proximal end 15.

**.** In accordance with an alternative embodiment, said ablation catheter 1 comprises an handle 103, wherein said steering device 144 is connected to said handle 103.

**.** In accordance with an alternative embodiment, said steering device 144 is drivable in rotation with respect to said handle 103 so that a rotation of said steering device 144 with respect to said handle causes a rotation of said elongate shaft 13.

**.** In accordance with an alternative embodiment, said steering device 144 comprises a through hole 145 in communication with said inner lumen 118.

**.** In accordance with an alternative embodiment, during insertion or removal of the shape setting mandrel 26 within or from said ablation catheter 1 said shape setting mandrel 26 passes through said through hole 145, and wherein when the shape setting mandrel 26 is fully inserted in the shaft distal portion 17, said mandrel proximal portion 138 is outside said steering device 144.

**.** In accordance with an alternative embodiment, when the shape setting mandrel 26 is fully inserted in the shaft distal portion 17, said shape setting mandrel 26 deforms said shaft distal portion 17 at least in a shaft distal portion plane P.

**.** In accordance with an alternative embodiment, said steering device 140 comprises at least two protrusion 147, wherein said at least two protrusions and said shaft distal portion plane P are coplanar to help a user to handle the catheter assembly 1.

**.** In accordance with an alternative embodiment, said ablation assembly 100 comprises a distal ablation assembly 21 disposable at least at said shaft distal portion distal end 19.

**.** In accordance with an alternative embodiment, said distal ablation assembly 21 being configured to deliver both thermal energy for ablating said tissue 41 and non-thermal energy for treating said tissue 41.

**.** In accordance with an alternative embodiment, said distal ablation assembly 21 comprises at least an electrode tip 128 disposable at least at said shaft distal portion distal end 19.

**.** In accordance with an alternative embodiment, said shaft electrodes 127 are arranged along the shaft distal portion 17 spaced apart from each other.

**.** In accordance with an alternative embodiment, said shaft ablation assembly 20 is configured also to map a tissue 41.

**.** In accordance with an alternative embodiment, said electrode tip 128 has an external surface shaped to be atraumatic and resiliently biased in rounded configuration.

**.** In accordance with an alternative embodiment, said shaft electrodes 127 and said electrode tip 128 comprise at least a monopolar electrode 113 and/or at least a bipolar electrode 114.

**.** In accordance with an alternative embodiment, said distal ablation assembly 21 comprises at least one thermocouple 37.

**.** In accordance with an alternative embodiment, said shaft ablation assembly 20 comprises at least one thermocouple 37.

**.** In accordance with an alternative embodiment, the shaft electrodes 127 are five to ten electrodes fixedly attached to the shaft distal portion 17.

**.** In accordance with an alternative embodiment, said electrode tip 128 is fixedly disposed at least at said shaft distal portion distal end 19.

**.** In accordance with an alternative embodiment, said electrode tip 128 is removable from said shaft distal portion distal end 19 and interchangeable with a set of tip electrodes 39, wherein the tip electrodes of the set of tip electrodes 39 have different shapes and dimensions.

**.** In accordance with an alternative embodiment, the shaft electrodes 127 are arranged spaced apart along a length of the shaft distal portion 17 in one of the following configurations:
spaced apart 1-5 cm, and/or
spaced apart 2-3 cm, or
spaced about 2-5mm apart, preferably 4mm apart, when a tension of 5000 volts is applied; or
spaced more than 5mm apart when a tension up to 5000 volts is applied;
   and/or
wherein each shaft electrode of said plurality of shaft electrodes 127 comprises an exposed length of up to 20-25 mm or 2-4 mm.

**.** In accordance with an alternative embodiment, each shaft electrode of said plurality of shaft electrodes 127 comprises an electrode surface area from about 0.05cm² to about 5cm² or from about 1cm² to about 2cm².

**.** In accordance with an alternative embodiment, said plurality of shaft electrodes 127 comprise a distal shaft electrode 106, said distal shaft electrode 106 being mounted on the shaft distal portion 17 at a distance of 2-4 mm from the shaft distal portion distal end 19.

**.** In accordance with an alternative embodiment, the shaft electrodes 127 are cylindrical.

**.** In accordance with an alternative embodiment, the shaft electrodes 127 have a profile that is flush with the surface of the shaft.

**.** In accordance with an alternative embodiment, the shaft electrodes 127 present a shaft electrodes outer diameter 36, and the shaft portions between the shaft electrodes 127 present an outer shaft diameter 35 that is slightly smaller than the shaft electrodes outer diameter 36 such that the shaft distal end is more flexible.

**.** In accordance with an alternative embodiment, the shaft electrodes 127 are resiliently biased in circular configuration.

**.** In accordance with an alternative embodiment, the shaft electrodes 127 present a tubular geometry having a wall thickness to outer diameter that approximates a 1:15 ratio.

**.** In accordance with an alternative embodiment, said plurality of shaft electrodes 127 comprise at least a bipolar electrode 114, said bipolar electrode 114 comprising a small electrode 130 and a large electrode 131, wherein the small electrode 130 is isolated from the large electrode 131.

**.** In accordance with an alternative embodiment, the shaft distal portion distal end 19 is open and the shape setting mandrel 26 is slidable outside said shaft distal portion distal end 19 from said mandrel fully inserted position to a mandrel maximum exposed position.

**.** In accordance with an alternative embodiment, said distal ablation assembly 21 is fixedly disposed at said mandrel distal portion 139.

**.** In accordance with an alternative embodiment, said distal ablation assembly 21 comprises a plurality of mandrel electrodes 132, wherein said mandrel electrodes 132 are axially spaced along said mandrel distal portion 139.

**.** In accordance with an alternative embodiment, said mandrel electrodes 132 comprise at least a monopolar electrode 113 and/or at least a bipolar electrode 114.

**.** In accordance with an alternative embodiment, when said shape setting mandrel 26 is in said mandrel fully inserted position, the shaft electrodes 127 are electrically connected with at least a part of the plurality of mandrel electrodes 119.

**.** In accordance with an alternative embodiment, when said shape setting mandrel 26 is in said mandrel maximum exposed position the shaft electrodes 127 are electrically disconnected from any electrical source.

**.** In accordance with an alternative embodiment, the shape setting mandrel 26 is slidable outside the shaft distal portion distal end 19 from a mandrel fully inserted position to a mandrel maximum exposed position. In said mandrel fully inserted position, the mandrel 26 is in said loaded straight configuration, and in said mandrel maximum exposed position, the mandrel is in said pre-shaped configuration.

**.** The present invention refers also to an ablation kit 200.

**.** Said ablation kit 200 comprises:
- at least an ablation equipment 100 according to any one of the preceding embodiments;
- a set of shape setting mandrels 134.

**.** The shape setting mandrels of said set 134 have different pre-shaped configurations.

**.** The shape setting mandrels of said set 134 are alternatively disposable and removable in said ablation catheter 1.

**.** According to an alternative embodiment, said set of shape setting mandrels 134 comprises at least a first shape setting mandrel 135 and a second shape setting mandrel 136.

**.** The first shape setting mandrel 135 has a first pre-shaped configuration and the second shape setting mandrel 136 has a second pre-shaped configuration.

**.** Said first pre-shaped configuration is different than said second pre-shaped configuration so that different shapes of shaft distal portion 17 are performed depending on which shape setting mandrel 135, 136 of said set of setting mandrels 134 is disposed into the ablation catheter 1.

**.** In accordance with an alternative embodiment, at least one shape setting mandrel of said set of shape setting mandrels 134, has a circular pre-formed configuration.

**.** In accordance with an alternative embodiment, at least one shape setting mandrel of said set of shape setting mandrels 134, has a spiral pre-formed configuration.

**.** In accordance with an alternative embodiment, at least one shape setting mandrel of said set of shape setting mandrels 134 has a straight pre-formed configuration.

**.** In accordance with an alternative embodiment, at least one shape setting mandrel of said set of shape setting mandrels 134 has a circular pre-formed configuration provided with an elbow.

**.** The present invention furthermore refers to ablation catheter Kit 300.

**.** The ablation catheter kit 300 comprises at least a first ablation assembly 100 and a second ablation assembly 100' according to any of the preceding described embodiments.

**.** The shaft distal portion 17 of the ablation catheter 1 of the first ablation assembly 100 is deflectable in at least two symmetric geometries.

**.** The shaft distal portion 17' of the ablation catheter 1' of the second ablation assembly 100' is deflectable in in at least two asymmetric geometries.

**.** Thanks to the solutions proposed, it is possible to provide a method for set shaping an ablation catheter, comprising the following steps:
- providing an ablation equipment 100 according to anyone of the above described embodiments,
- inserting said shape setting mandrel 26 in said loaded straight configuration within said inner lumen 118 of said ablation catheter 1,
- moving said shape setting mandrel 26 within said inner lumen 118 towards the shaft distal portion distal end 19 until the shape setting mandrel 26 is fully inserted into said shaft distal portion 17, and
- conforming the shape of shaft distal portion 17 to the pre-shaped configuration of said shape setting mandrel 26 when the shape setting mandrel 26 is fully inserted into said shaft distal portion 17.

**.** Thanks to the solutions proposed, it is possible to provide a method for the treatment of proximal, persistent or long-standing persistent atrial fibrillation in a patient comprising the following steps:
- providing an ablation equipment 100 according to anyone of the above described embodiments;
- placing the ablation catheter 1 in the coronary sinus of the patient, such as to map electrograms and/or to deliver both non-thermal energy for treating a tissue and thermal energy for ablating a tissue 41, and subsequently;
- place the ablation catheter 1 in the left or right atrium to map electrograms and/or to deliver both nonthermal energy for treating a tissue 41 and thermal energy for ablating a tissue 41 ,
   wherein the tissue locations include fasicals around a pulmonary vein, and/or the left atrial roof, and/or the mitral isthmus.

**.** Thanks to the solutions proposed, it is possible to provide a method for the treatment of atrial flutter in a patient comprising the following steps:
**.** - providing an ablation equipment 100 according to anyone of the above described embodiments;
**.** - placing the ablation catheter 1 in one or more locations in the right atrium of the heart 43 to achieve bi-directional block by delivering both non-thermal energy for treating a tissue 41 and thermal energy for ablating a tissue 41.

**.** Thanks to the solutions proposed, it is possible to provide a method of ablating tissue in the right atrium of the heart 43 comprising the following steps:
- providing an ablation equipment 100 according to anyone of the above described embodiments;
- placing the ablation catheter 1 in one or more locations in the right and/or left atrium of the heart 43; - creating lesions between the superior vena cava and the inferior vena cava and/or the coronary sinus and the inferior vena cava and/or the superior vena cava and the coronary sinus by delivering both non-thermal energy for treating a tissue 41 and thermal energy for ablating a tissue 41.

**.** Thanks to the solutions proposed, it is possible to provide a method for the treatment of sinus node tachycardia in a patient comprising the following steps:
- providing an ablation equipment 100 according to anyone of the above described embodiments;
- placing the ablation catheter 1 in one or more locations in the right and/or left atrium of the heart 43;
- mapping electrograms sinus node and/or mapping sinus node and/or ablating the sinus node by delivering both non-thermal energy for treating a tissue and thermal energy for ablating a tissue.

**.** Thanks to the solutions proposed, it is possible to provide a method for the treatment of ventricular tachycardia in a patient comprising the following steps:
- providing an ablation equipment 100 according to anyone of the above described embodiments;
- placing the ablation catheter 1 in the left or right ventricles of the heart 43;
- inducing ventricular tachycardia by delivering pacing energy, and
- ablating tissue to treat the patient by delivering both non-thermal energy for treating a tissue 41 and thermal energy for ablating a tissue 41.

**.** Thanks to the solutions proposed, it is possible to provide a method to ablate atrial tissues comprising the following steps:
- providing an ablation equipment 100 according to anyone of the above described embodiments;
   wherein the shaft distal portion 17 comprises a first deflection geometry when the shape setting mandrel 26 is fully inserted in the elongate shaft 13, and the shaft distal portion 17 comprises a second deflection geometry when the shape setting mandrel 26 is removed from the shaft distal portion 17, wherein the first deflection geometry is larger than the second deflection geometry;
- placing the ablation catheter 1 exposed to an atrial tissue, with the shaft distal portion 17 in the second deflection geometry with said shape setting mandrel 26 outside said distal portion 17;
- ablating one or more of the following tissue locations: left atrial septum; tissue adjacent the left atrial septum; and tissue adjacent the left atrial posterior wall by delivering both non-thermal energy for treating a tissue and thermal energy for ablating a tissue;
- placing the ablation catheter 1 with the shaft distal portion 17 in the first deflection geometry by fully inserting the shape setting mandrel 26 within the elongate shaft 13,
- ablating at least the circumference around the pulmonary veins by delivering both non-thermal energy for treating a tissue 41 and thermal energy for ablating a tissue 41.

**.** Referring to the figures, one embodiment of an energy delivery system for selectively ablating tissue, or ablation equipment or assembly 100, is illustrated. In one aspect, the system can comprise at least one energy delivery device, or ablation catheter 1, such as, but not limited to, a monopolar probe 101, and at least one energy delivery source or power source, or single power source, 4. In one aspect, at least a portion of the probe can be configured for insertion into a patient. In one aspect, the at least one energy source, or single power source 4, can further comprise at least a non-thermal energy source 6 and a thermal energy source 7. In one aspect, the system can comprise a mechanism for coupling the probe to one desired energy source of the at least one energy source 8, or probe connector. In one aspect, although a monopolar probe is described herein, one of ordinary skill in the art will recognize that the energy delivery device used with the system described herein can be a different type of energy delivery device, such as, but not limited to, a bipolar probe 102. In one aspect, the probe can be selected from a group consisting of: a monopolar electrode 113, a bipolar electrode 114, and an electrode array 111, such as shaft electrodes 127, mandrel electrodes 132, and tip electrode 128.

**.** This can allow for utilization of an optimal energy delivery device for a given medical procedure. In one aspect, the monopolar probe 101 can comprise a handle 103, a electrode having a proximal end, or electrode proximal end 104, and a distal end, or electrode distal end 105, and at least one connector of the probe. In one aspect, the electrode(s) can comprise at least one distal electrode 106 that is positioned therein at the distal end of the probe and round electrodes 107 positions on the body of the probe that is positioned in the heart chamber. In one aspect, the tip can be a rounded conical type shape and can be capable of sliding along the wall of the heart and said probe designed to allow the sliding to match the heart wall motion.

**.** In one aspect, at least one monopolar probe, as described above, can be used with system. In another aspect, although not illustrated, at least two monopolar electrodes 113, as described above, can be used with system. In one exemplary embodiment, it is contemplated that if more than one electrode is used in the system, the probes can be used in various configurations and shapes, such as, but not limited to, a parallel configuration, a spiral configuration or an adjacent configuration. In one aspect, if two electrodes are used, it is contemplated that the distal electrode would be one and each (any one or more) of the catheters body electrodes would be selected based on the length requirements of the ablation. In another exemplary aspect, the electrodes can be positioned such that the distal tip can be staggered in length compared to a body electrode. In one exemplary embodiment, if at least two electrodes are used in the system, the at least two electrodes can be spaced about 2-5mm apart while mounted on the catheter body inserted into heart chamber and can provide a voltage of up to 5000 volts. In yet another exemplary embodiment, the at least two electrodes can be spaced about >5mm apart and be selecting alternate electrodes on catheter body and can have a voltage of up to about 5000 volts. In one exemplary embodiment, the at least two electrodes can be spaced from each other such that they are approximately 4mm apart while inserted into a target tissue and can provide a voltage of up to approximately 5000 volts.

**.** The at least one electrode of the monopolar probe can be configured to be electrically coupled to and energized by energy source. Further, although not shown, one of ordinary skill in the art would recognize that at least one patient return pad 108 can be used in conjunction with the at least one electrode to complete an electrical circuit 109. Although a single electrode configuration is described herein, it is contemplated that other various needle 110 and/or electrode array formations could be used in any of the embodiments described herein. In one aspect, this array could be a plurality or series of monopolar and/or bipolar probes arranged in various shapes, configurations, or combinations in order to allow for the ablation of multiple shapes and sizes of target regions of tissue. Various array patterns can reduce the need to reposition the electrode array during treatment by allowing multiple selectively activatable electrode patterns 112. In one aspect, the electrodes can be of different sizes and shapes, such as, but not limited to, square, oval, rectangular, circular or other shapes. In one aspect, the electrodes described herein can be made of various materials known in the art.

**.** In one aspect, the electrodes described herein can be exposed up to various lengths. In one aspect, the electrodes can have an exposed length of up to approximately 20 - 30mm placed onto cardiac tissue, such can be either linear length or circular length as in the case where the at least two electrodes are spaced up to approximately 2-5mm apart on catheter body and distal tip. In another exemplary aspect, the electrodes can have an exposed electrode length of up to approximately 2-4mm, such as in the case where the at least two electrodes are spaced approximately 2-5mm apart. In yet another aspect, the electrodes can be spaced greater then 4mm distances from one another. In one aspect, the electrodes can be spaced apart a distance of from about 0.4cm to about to 1cm. In another exemplary embodiment, the electrodes can be spaced apart a distance of from about 1 cm to about 5 cm. In yet another embodiment, the electrodes can be spaced apart a distance of >2cm. In one exemplary aspect the electrode surface area can vary. In one exemplary embodiment, the electrode surface area can vary from about 0.05cm² to about 5cm². In yet another exemplary embodiment, the electrodes can have a surface area of between about 1cm² to about 2cm².

**.** In one aspect, the system can comprise a means 11, 12 for selectively energizing a desired energy source to ablate at least a portion of the tissue adjacent to the at least one probe. In one aspect, the non-thermal energy source 6 of the at least one energy source or single power source 4, can be selectively energized to apply non-thermal energy to at least a portion of the desired tissue region to ablate at least a portion of the desired tissue region 45. Thus, in one aspect, the energy source can be configured to deliver non-thermal energy, such as, but not limited to, electroporation energy to target tissue. In one exemplary embodiment, the thermal energy source can be an RF energy source. In one aspect, although not shown, during use of the system, the at least one electrode / probe can be selectively coupled to either of the non-thermal energy source or the thermal energy source, and the desired energy source can be selectively energized to apply either nonthermal, thermal or both energies from the selected energy source to at least a portion of the desired tissue region to ablate at least a portion of the desired tissue region In one exemplary aspect, the at least one energy source can have at least one connector 8 that is configured for selective coupling to the at least one electrode / probe. In one aspect, the energy source can have a positive connector 9 and a negative connector 10. More particularly, the at least one connector of the electrode / probe can be connected to the energy source via at least one of the positive connector and the negative connector.

**.** In one exemplary embodiment, the power source or energy source can be a electrosurgical generator capable of delivering both thermal and non-thermal energies. In one aspect, the battery power supply can be capable of being manually adjusted, depending on the voltage or automatically. In one aspect, at least one of the power outlets, generators, and battery sources described herein can be used to provide voltage to the target tissue during treatment. The battery powered energy source is more than 95% efficient in converting the battery power into RF or high voltage pulsed fields.

**.** In one aspect, the cardiac arrhythmia ablation system is fully electrically Isolated and no leakage current due to the battery design, no connection to the power grid or earth ground.

**.** In yet another exemplary embodiment, to achieve IRE ablation of the target region of tissue, the power source or generator can be used to deliver IRE energy to target tissue, including target tissue that can be somewhat difficult to reach. In one aspect, an exemplary embodiment of an IRE generator can include anywhere from 2 to 10 positive and negative connectors, though one of ordinary skill in the art would understand that other numbers of positive and negative connectors and different embodiments of connectors could be used and may be and necessary for optimal ablation configurations. Whereas, output power controlled using either amplitude or duty cycle or both, Defib protected up to 10kv.

**.** A system in which a bipolar probe 102 is used. In one aspect, the bipolar probe 102 can comprise a handle 103, electrode having a proximal end 104 and a distal end 105, and at least one probe connector 9. In one aspect, the electrode can comprise at least one electrode that is positioned therein at the distal end of the catheter and that is positioned at a distal most portion of the ablation elements. In one aspect, the electrode can further comprise a first electrode 115 that is positioned at the distal most portion of the catheter, a second electrode 116 that is positioned proximal of the distal electrode, and at least one spacer 117 that can be positioned between and adjacent to at least a portion of each of the first and second electrodes and the third, etc. electrode. In one aspect, at least a portion of a distal portion of the second electrode can abut at least a proximal portion of spacer and at least a distal portion of spacer can abut at least a portion of a proximal portion of the first electrode. In one aspect, similar to monopolar probe, the bipolar probe can be coupled to either type energy source 8. During use of the system, the probe can be coupled to the energy source. More particularly, in one exemplary aspect, at least one connector of the probe 8 can be connected to the energy source via at least one of the positive connectors 9 and the negative connector 10, as also described above.

**.** Nonthermal IRE ablation involves ablation where the primary method of cellular disruption leading to death is mediated via electroporation (rather than factors such as effects of or responses to heating). In certain embodiments, depending on the parameters mentioned (including time that the resulting temperature occurs), cellular death can be mediated via nonthermal IRE up to approximately >46 degrees C. In certain embodiments cellular damage from thermal heating occurs above approximately >46 degrees C. In various embodiments, the parameters resulting in nonthermal IRE can be changed to result in the death of cells via thermal heating. The parameters can also be changed to from one having nonthermal IRE effects to alternative settings where the changed parameters also have nonthermal IRE effects.

**.** More particularly, in one aspect, the total number of pulses of pulse trains 204 in various embodiments can be varied based on the desired treatment outcome and the effectiveness of the treatment for a given tissue. During delivery of non-thermal electroporation, the preferred means to achieve the high voltage pulsed fields would be using a sinewave. Previous literatures supports using a squarewave, similar to that of a DC pulsed field. The issue with squarewave pulsed electric fields are the similarities to that of an ICD (internal cardiac defibrillator), these types of devices cause significant heart tissue damage when discharged. For squarewave Pulsed Electric Field ablation, causing heart tissue damage outside the desired zone is problematic. As such, sedation is required and square delivery must be timed with the R-wave of the ECG. This is all due to the negative effects of square wave pulsed electric field ablations. Sinewave pulsed electric field ablation does not have these characteristics and thus do not cause heart tissue damage outside the ablation zone, do not cause pain, do not need to be delivered during any particular portion of the ECG.
IRE energy to target tissue, a voltage can be generated that is configured to successfully ablate tissue and using sinewave energies, do not cause unwanted damages. In one aspect, certain embodiments can involve pulses between about 1 microsecond and about 80,000 milliseconds, while others can involve pulses of about 75 microseconds and about 20,000 milliseconds. In yet another embodiment, the ablation pulse applied to the target tissue 47 can be between about 20 microseconds and 100 microseconds. In one aspect, the at least one energy source can be configured to release at least one pulse of energy for between about 100 microseconds to about 100 seconds.
In certain embodiments the electrodes described herein can provide a voltage of about 100 volts per centimeter (V/cm) to about 7,000 V/cm to the target tissue. In other exemplary embodiments, the voltage can be about 200 V/cm to about 2000 V/cm as well as from about 300 V/cm to about 1000 V/cm. Other exemplary embodiments can involve voltages of about 2,000 V/cm to about 20,000 V/cm. In one exemplary aspect, the bipolar probe 100 can be used at a voltage of up to about 2700 volts.

**.** In one exemplary aspect, at least two monopolar electrodes 113 can be used to ablate target tissue, while at the same time, at least two bipolar electrodes can be used. The aforementioned is selected based on patient anatomy, disease state and optimal therapeutic needs of the patient. In one exemplary embodiment, two single electrodes can be configured so as to involve other ablation areas. One of ordinary skill in the art would be understood that the ablation size, shape and depth requirement can be advantageously varied with placement of the electrode and various electrode selected and the type of energy selected. In one aspect, during treatment, an additional area surrounding an outer edge of the target region of tissue is also ablated (ablation of unwanted or diseased tissue). This surrounding area of tissue can be ablated in order to ensure patient safety and the complete and adequate ablation of the target region of tissue. In one aspect, during the method of use, the catheter electrode tip 128 of the catheter is designed as not to puncture a patient's tissue. One of ordinary skill in the art would recognize that the target region of tissue can be any tissue from any organ where ablation can be used to ablate unwanted or diseased tissue, such as, but not limited to, cardiac tissue, digestive, skeletal, muscular, nervous, endocrine, circulatory, reproductive, integumentary, lymphatic, urinary tissue or organs, or other soft tissue or organs where selective ablation is desired. Soft tissue can include, but is not limited to, any tissue surrounding, supporting, or connecting other body structures and/or organs. For example, soft tissue can include muscles, tendons, ligaments, fascia, joint capsules, and other tissue. More specifically, target tissue can include, but is not limited to, areas of the heart, the prostate (including cancerous prostate tissue), the kidney (including renal cell, carcinoma tissue), as well as breast, lung, pancreas, uterus, and brain tissue, among others.

**.** In one aspect, the energy source can be a thermal energy source and/or in one aspect, the nonthermal energy source which are both sinewave generated energies can be selectively energizing for a desired period of time. More particularly, the period of time can be a predetermined period of time. In yet another aspect, the period of time can be a plurality of predetermined periods of time. In one aspect, the thermal energy source is selected from the group consisting of radiofrequency (RF), focused ultrasound, microwave, lasers, thermal electric heating, traditional heating methods with electrodes using DC or AC currents, and the application of heated fluids and cold therapies (such as cryosurgery). RF energy is known in the art for effective use in tumor ablation, though it is clear that any form of temperature-mediated continuous ablation could be used at settings known the art. In one aspect, after the energy delivery device is inserted into target organ 44, tissue 43 is ablated, and the energy delivery device is withdrawn. In one aspect the thermal energy source 7 can be an alternating current thermal energy source. In yet another aspect, the thermal energy source 7 is a direct current thermal energy source.

**.** In one aspect, the electrode(s) can start at the point of non-thermal ablation of the target region. In one aspect, thermal ablation can be initiated at the start of the electrode chain (length wise on the catheter), which in one embodiment is applied to prevent aberrant tissue conduction. As the energy delivery device or electrode is withdrawn, thermal energy can be applied through the electrode to the target tissue. In one aspect, the electrode is selectively energized with thermal energy or nonthermal to ablate tissue adjacent the electrode track and proximate to a boundary of the tissue ablated.

**.** In one aspect, IRE treatment of target tissue, followed by thermal ablation of at least one tissue area can be performed during procedures such as, but not limited to, cardiac, laparoscopic procedures and open surgical procedures. In one aspect ablation track can be ablated during the repositioning or dragging of a electrodes. In one aspect, after delivery of IRE energy to the target tissue, an ablated region of tissue remains. In one aspect, ablated region of tissue includes target tissue region and the surrounding area of tissue. In one exemplary embodiment, after treatment of the target tissue using IRE, treatment parameters can be reset to bring about thermal track ablation. In one aspect, after IRE treatment of the target tissue, the energy delivery device or electrodes is repositioned. In one aspect, upon termination of the energy delivery (and in some cases repositioning) of the energy delivery device ablate tissue in a different area/location, a tissue track is coagulated and bleeding can be prevented. In one aspect thermal energy, such as, but not limited to RF energy, can be applied to the ablation track during the ablation cycle. In another aspect the track ablation zone is created to stop bleeding. It is important to prevent bleeding so as no clots are formed, especially during procedures that could involve ablation in the left-side of the heart.
In one aspect, the generator, or single power source 4, used during the thermal ablation procedure can be configured to have various ablation settings and capabilities. In one exemplary aspect, the Arga generator described above can be used as an RF energy source. In one aspect, the RF energy source can be used to ablate tissue using 10-1000 watts of power, either duty-cycled or steady delivery. In other exemplary aspects, one of ordinary skill in the art would recognize that smaller or larger amounts of power can be used in various embodiments, as necessary, in order to provide ablation. In one exemplary embodiment utilizing the generator, the RF power source can provide AC power in addition to being used for ablation, while the IRE power source can be used to provide DC power.

**.** In one aspect, if a thermal energy source is used, it could be used with a variety of techniques to bring about tissue ablation. In one exemplary aspect, additional embodiments can involve ablation performed using one or more of radiofrequency (RF), focused ultrasound, microwaves, lasers, thermal electric heating, traditional heating methods with electrodes using DC or AC currents, and application of heated fluids and cold therapies, such as, but not limited to, that used in cryosurgery. In one aspect the heat energy can be delivered in certain embodiments via pulses that can be in a range of about 35 microseconds to about 10 seconds. In other exemplary embodiments the at least one energy source can be configured to release or deliver at least one pulse of heat energy in a range of about 35 microseconds to about 1 second. In yet another exemplary embodiment, at least one energy source can release or deliver at least one pulse of energy for between about 35 microseconds to about 1000 microseconds. In yet another exemplary embodiment, at least one pulse can be delivered in a range of from about 1 microsecond to about 100 microseconds.

**.** In one exemplary embodiment thermal energy can be applied such that it produces fluctuations in temperature to effect treatment. In one aspect, the thermal energy provided to the tissue can heat the target tissue to between about 46 degree C and about 70 degrees C to bring about cell death. In one aspect the temperature can be adjusted such that it can be lesser or greater than this temperature range, depending on the exact rate of speed of removal of the heat generated via externally supplied fluid and/or blood from the target tissue. In one embodiment the temperature used is between about 50 degrees C and about 100 degrees C, although one of ordinary skill would recognize that temperatures above about 100 degrees C can cause tissue vaporization. Ellis L, Curley S, Tanabe K. Radiofrequency Ablation for Cancer; Current Indications, Techniques, and Outcomes, NY: Springer, 2004. In one exemplary embodiment, thermal energy can be used to ablate approximately 2-3 mm of tissue. In one aspect this tissue thickness can be varied depending upon various factors, such as, but not limited to, the condition of the target tissue, the various parameters used, and the treatment options.

**.** In one embodiment the mechanisms through which the user sets the parameters for bringing about the desired ablation effects are changed to bring about either thermal results through thermal heating that is resistive heating or non-thermal by high voltage pulsed electric fields. In certain embodiments the mechanisms are reset such that sinewave energy is applied to bring about thermal ablation or non-thermal ablations. In one exemplary embodiment, ablation can be performed using sinewave current. In one aspect, the sinewave current can be used for heating the target tissue. In one aspect, at least one pulse of sinewave current can be delivered in one direction. In yet another aspect, at least one pulse of sinewave current can be delivered from the opposite direction of an electrical circuit. In one aspect, sinewave current can be applied such that the temperature of the tissue can be between about 42 degrees C and about 75 degrees C. In one aspect, the sinewave current can be applied such that thermal damage is induced at a temperature as low as about 42 degrees C.

**.** One of ordinary skill in the art would recognize that various lengths of sinewave pulses, amplitude of sinewave pulses can be varied and applied to bring about effective ablation of the non-thermal and the thermal type from the same system or of different systems. In summary, the method for selectively ablating tissue involves providing at least one sinewave energy source, such as a sinewave generator, described above. In one aspect, the at least one energy source, or single power source 4, can comprise at least a non-thermal energy source 6 and a thermal energy source 7, providing at least one probe, or at least one ablation catheter 1, that is configured to be selectively manually operatively coupled to a desired energy source of the at least one energy source, positioning, via a electrode, at least a portion of the at least one electrode within a desired region of a target tissue. In one aspect, the selective coupling of the electrodes to the thermal energy source comprises the actuating a switch 40 to operatively select between the non-thermal energy source 7 and the thermal energy source 8. In one aspect, the selective coupling of the electrodes to either or both monopolar and/or bipolar energy source comprises tailoring of the therapeutics effects to the patient. Then at least one probe is selectively coupled to the non-thermal energy source(monopolar and/or bipolar), and the non-thermal energy source is selectively energized to apply non-thermal energy from the non-thermal energy source to at least a portion of the desired region to ablate at least a portion of the desired region, selectively coupling the at least one probe to the thermal energy source, if desired. In one aspect, prior to selectively coupling the at least one probe to the desired energy source and determining the optimal monopolar, bipolar delivery method, the at least one probe is operatively connected to a ECG recording and mapping system to view and analyze the hearts electrical conduction.

### . References

**.** Mali B, Jarm T, Snoj M, Sersa G, Miklavcic D. Antitumor effectiveness of electrochemotherapy: A systematic review and meta-analysis. Eur J Surg Oncol. 2013;39:4-16.
**.** Heller R, Heller LC. Gene Electrotransfer Clinical Trials. Adv Genet. 2015;89:235-62.
**.** Neumann E, Schaefer-Ridder M, Wang Y, Hofschneider P. Gene transfer into mouse lyoma cells by electroporation in high electric fields. EMBO J. 1982;1:841-5.

### LIST OF REFERENCE NUMERALS

- 1: ablation catheter OR energy delivery system OR energy delivery device OR probe OR multi-electrode and multi-functional ablation catheter
- 3: system for selectively ablating tissue
- 4: single power source OR energy source OR energy delivery source OR generator
- 5: battery powered generator
- 6: non-thermal energy source
- 7: thermal energy source OR alternating current thermal energy source OR direct current thermal energy source
- 8: means for selectively coupling the probe to one desired energy source of the at least one energy source OR mechanism for coupling the probe to one desired energy source OR probe connector
- 9: positive connector
- 10: negative connector
- 11: means for selectively energizing the non-thermal energy source
- 12: means for selectively energizing the thermal energy source
- 13: elongate shaft
- 14: elongate shaft proximal portion
- 15: elongated shaft proximal end
- 16: elongate shaft distal end
- 17: elongated shaft distal portion
- 18: elongated shaft distal portion proximal end
- 19: elongated shaft distal portion distal end
- 20: shaft ablation assembly OR functional element fixedly mounted to the distal portion
- 21: distal ablation assembly OR tip ablation element OR Tip OR mandrel with electrodes
- 22: shaft ablation element OR electrode OR single/multiple ablation element
- 23: tip ablation element
- 24: deflection shapes and geometries of the distal portion OR deflection geometries
- 25: steering wire (configured to deflect the distal portion in the one or more deflection directions)
- 26: shape setting mandrel OR deflection assembly (to maintain deflections in a single plane)
- 27: asymmetric joint (between two elongate shaft portions)
- 28: integral member
- 29: variable braid OR steering wires
- 30: control port OR aperture on the tip of the elongate shaft
- 31: single ablation element OR ablation element (suitable for RF and Irreversible Electroporation) OR electrode
- 32: multiple ablation elements OR electrodes
- 33: shape setting mandrel carrier assembly OR shape setting mandrel OR deflection assembly OR mandrel
- 34: control shaft OR proximal portion of the mandrel
- 35: shaft outer diameter
- 36: ablation electrodes / ablation elements outer diameter
- 37: thermocouple
- 38: means of dissipating heat (such as increased surface area)
- 39: set of electrode tips
- 40: switch to operatively select between the non-thermal energy source and the thermal energy source
- 41: tissue
- 42: ablated tissue
- 43: heart
- 44: organ
- 45: ablating region OR desired region
- 100: ablation equipment or assembly
- 101: monopolar probe OR ablation catheter having monopolar solution OR ablation catheter having a monopolar arrangement of the at least one electrode
- 102: bipolar probe OR ablation catheter having a bipolar arrangement of the electrodes
- 103: handle
- 104: electrode proximal end
- 105: electrode distal end
- 106: distal electrode
- 107: round electrodes
- 108: grounding pad
- 109: electrical circuit
- 110: needle
- 111: electrode array OR orderly arrangement of multiple probes
- 112: multiple selectively activatable electrode patterns.
- 113: monopolar electrode
- 114: bipolar electrode
- 115: first electrode OR most distal portion electrode
- 116: second electrode OR proximal electrode
- 117: spacer
- 118: inner lumen (2nd Lumen - multi-purpose (fluid flush and shape setting mandrel))
- 119: mandrel elastic body
- 120: catheter bend portion
- 121: mandrel heating element
- 122: mandrel locking mechanism
- 123: retention element
- 124: locking seat
- 125: ball-tip
- 126: shaft transition portion
- 127: shaft electrodes
- 128: electrode tip /atraumatic tip
- 130: small electrode
- 131: large electrode
- 132: mandrel electrodes
- 134: set of shape fitting mandrel
- 135: first shape setting mandrel
- 136: second shape setting mandrel
- 138: mandrel proximal portion
- 139: mandrel distal portion
- 140: mandrel seat
- 141: inner lumen neck portion
- 142: wire proximal extension
- 143: wire gripping portion
- 144: steering device
- 145: steering device through hole
- 200: Kit of ablation catheter and set of mandrels
- 204: pulse train
- 207: catheter elongated shaft flexible body = flexible body
- 208: body vessels
- 210: wire
- 300: kit of ablation catheters
- 400: single control unit
- 401: power unit
- 402: power module
- 403: drive circuit block
- 404: selecting block
- 405: filtering block
- 406: electrical isolation block
- 407: Microprocessor
- 408: variable High Voltage Power Supply block
- 409: Programmable Logic Controller block
- 410: Video interface block
- 411: Watch Dog block
- 412: Audio interface block
- S: sinusoidal electric signal
- Vcc: supply voltage signal
- N: insulated conductive portions of an electrode
- IRE: irreversible electroporation
- RF: radiofrequency
- X-X: elongate shaft longitudinal main direction
- P: shaft distal portion plane
- T: time interval
- T1: first time interval
- ALFA: acute angle
- 410': Push Button block
- 114a: first electrode
- 424: electrode body
- 114b: second point-like electrode
- 210a: first wire
- 210b: second wire
- 425: ground electrode

## Claims

1. Ablation equipment (100) to treat target regions of tissue (41) in organs (44), comprising:
an ablation catheter (1) and a power source (4);
said ablation catheter (1) comprising:
a catheter elongated shaft (13) comprising at least an elongated shaft distal portion (17);
said catheter elongated shaft (13) comprising a flexible body (207) to navigate through body vessels (208);
said ablation catheter (1) further comprising a shaft ablation assembly (20) disposed at said elongated shaft distal portion (17) and a distal ablation assembly (21) extendable through said shaft ablation assembly (20) and out from said elongated shaft distal portion (17) via a port (30) of said ablation catheter (1);
said shaft ablation assembly (2) comprising at least a first plurality of electrodes (127, 113 or 114) fixedly disposed at said elongated shaft distal portion (17);
said distal ablation assembly (21) comprising at least a second plurality of electrodes (132) fixedly disposed along said distal ablation assembly (21), said distal ablation assembly (21) configured to conform to a predefined shape such that the second plurality of electrodes (132) are arranged according to the predefined shape upon said distal ablation assembly (21) extending out from said elongated shaft distal portion (17) via said port (30); and
all electrodes of said at least the first plurality (127, 113 or 114) and the second plurality (132) being electrically powered by said power source (4) through an electric signal (S) to deliver non-thermal energy for treating the tissue (41) and thermal energy for ablating the tissue (41);
wherein said electric signal (S) comprises a sinusoidal wave,
wherein said electric signal (S) is supplied to the electrodes of said first plurality (127, 113 or 114) and said second plurality (132) during a time interval (T), and
wherein said electric signal (S) is a sinusoidal pulse train (204) comprising two or more basic sine waves (BSW) in said time interval (T), wherein each basic sine wave (BSW) consists of one positive half-wave and one negative half-wave.

2. The ablation equipment (100) according to claim 1, wherein said power source is single power source.

3. The ablation equipment (100) according to claim 1, wherein said power source (4) comprises a single control unit (400) and a power unit (401) for generating said electric signal (S) comprising a sinusoidal wave, and
wherein said power unit (401) is electrically connected to all electrodes of said first plurality of electrodes (127, 113 or 114) and said second plurality of electrodes (132).

4. The ablation equipment (100) according to claim 3, wherein said control unit (400) is configured to drive the power unit (401) to modify the number of pulses in the sinusoidal pulse train (204) to change the electric energy level associated to the electric signal (S).

5. The ablation equipment (100) according to claim 3, wherein said sinusoidal pulse train electric signal (S) comprises from two to twenty-five basic sine waves (BSW) in said time interval (T), and/or
wherein each basic sine wave (BSW) comprises a duration equal to a first-time interval (T1), and/or
wherein said control unit (400) is configured to drive the power unit (401) to modify the duration of the first-time interval (T1) of the basic sine wave (BSW) to change the electric energy level associated to the electric signal (S).

6. The ablation equipment (100) according to claim 3, wherein said electric signal (S) comprising a sinusoidal wave is a voltage signal, a peak-to-peak mean amplitude of each basic sine wave (BSW) is in the range of 1.000 V to 2.000 V.

7. The ablation equipment (100) according to claim 3, wherein said power unit (401) comprises a power module (402) comprising:
- a drive circuit block (403) controlled by the control unit (400) for generating said electric signal (S) starting from a supply voltage signal (Vcc) provided by the single control unit (400);
- a selecting block (404) selectively controlled by said drive circuit block (403) to change continuously the electric energy level associated to said signal (S); and
- a filtering and electrical isolation block (405, 406).

8. The ablation equipment (100) according to claim 3, wherein said control unit (400) comprises:
a Microprocessor (407) configured to control a variable High Voltage Power Supply block (408) and a Programmable Logic Controller block (409);
said variable High Voltage Power Supply block (408) being configured to provide said supply voltage signal (Vcc) to the power module (402) for generating said electric signal (S);
said Programmable Logic Controller block (409) being configured to generate drive signals to control the drive circuit block (403) of the power module (402); and
said single control unit (400) further comprising:
- a Video interface and Push Button block (410, 410') controlled by the Microprocessor (407) to set parameters of the equipment (100) and display the selected parameters;
- a Watch Dog block (411) for controlling proper functioning of the Microprocessor (407); and
- an Audio interface block (412) for providing audio information representative of correctness of the ablation process and/or errors occurred.

9. The ablation equipment (100) according to claim 1, wherein
said ablation catheter (1) comprising said catheter elongated shaft (13) having a longitudinal main direction (X-X), said catheter elongated shaft (13) comprising said elongated shaft distal portion (17), said elongated shaft distal portion (17) comprising a shaft distal portion distal end (19);
said ablation catheter (1) comprising an inner lumen (118) arranged within the catheter elongated shaft (13) that is configured to receive the distal ablation assembly (21);
said ablation catheter (1) comprising:
- said shaft ablation assembly (20) fixedly disposed at said shaft distal portion (17), the shaft ablation assembly (20) being configured to deliver both thermal energy for ablating said tissue (41) and non-thermal energy for treating said tissue (41); and
- at least a shape setting mandrel (26) disposed on said distal ablation assembly (21) such that said shape setting mandrel (26) is moveable within the ablation catheter (1), the shape setting mandrel (26) being insertable within the inner lumen (118) and removable from the inner lumen (118),
wherein the shape setting mandrel (26) is free to move in respect of the inner lumen (118) avoiding any constraint with said elongated shaft distal portion (17) during the shape setting mandrel insertion,
wherein the shape setting mandrel (26) comprises at least a pre-shaped configuration and the shape setting mandrel (26) is reversibly deformable between at least a straight loaded configuration and said pre-shaped configuration,
wherein, when the shape setting mandrel (26) is fully inserted in the elongated shaft distal portion (17), the shape setting mandrel (26) is configured to shape set said elongated shaft distal portion (17) with said pre-shaped configuration.

10. The ablation equipment (100) according to claim 9, wherein said elongated shaft distal portion (17) is elastically deformable and/or wherein when the shape setting mandrel (26) is fully inserted in the elongated shaft distal portion (17), said elongated shaft distal portion (17) is configured to conform to said pre-shaped configuration.

11. The ablation equipment (100) according to claim 9, wherein when the shape setting mandrel (26) is fully inserted in the elongated shaft distal portion (17) it is defined a mandrel fully inserted position, and
wherein while the shape setting mandrel (26) slides within the inner lumen (118) towards said mandrel fully inserted position, and wherein the shape setting mandrel (26) is configured to variably shape set the elongated shaft distal portion (17) passing from said loaded straight configuration to said pre-shaped configuration.

12. The ablation equipment (100) according to claim 5, wherein said first-time interval (T1) is selected in the range of 1µsec to 80.000msec, or selected in the range of 75µsec-20.000msec, or selected in the range of 20µsec-100µsec.

13. The ablation equipment (100) according to claim 1, wherein the sinusoidal pulse train electric signal (S) is supplied to the electrodes (127, 113 or 114) during a time interval (T) selected in the range of 100µsec-100sec.

14. The ablation equipment (100) according to claim 1, wherein the electrodes of said at least the first plurality (127, 113 or 114) are electrically powered by said power source (4) to deliver a voltage to treat the target regions of tissue (41) which is selected in the range of 100 V/cm-7000 V/cm, or selected in the range of 200 V/cm-2000 V/cm, or selected in the range of 300 V/cm-1000 V/cm.

15. The ablation equipment (100) according to claim 1, wherein at least one electrode of said first plurality of electrodes (127) comprises two conductive portions (N) electrically isolated from each other and/or wherein at least one electrode of said first plurality of electrodes (127) comprises four conductive portions (N) electrically isolated from each other.

## Patentansprüche

1. Ablationsvorrichtung (100) zum Behandeln von Zielbereichen von Gewebe (41) in Organen (44), umfassend:
einen Ablationskatheter (1) und eine Stromquelle (4);
wobei der Ablationskatheter (1) umfasst:
einen länglichen Katheterschaft (13), der mindestens einen distalen Abschnitt des länglichen Schafts (17) umfasst; wobei
der längliche Katheterschaft (13) einen flexiblen Körper (207) zum Navigieren durch Körpergefäße (208) umfasst;
der Ablationskatheter (1) ferner umfasst: eine Schaft-Ablationsanordnung (20), die an dem distalen Abschnitt des länglichen Schafts (17) angeordnet ist, und eine distale Ablationsanordnung (21), die durch die Schaft-Ablationsanordnung (20) und aus dem distalen Abschnitt des länglichen Schafts (17) über eine Öffnung (30) des Ablationskatheters (1) herausziehbar ist;
die Schaft-Ablationsanordnung (2) mindestens eine erste Vielzahl von Elektroden (127, 113 oder 114) umfasst, die fest an dem distalen Abschnitt des länglichen Schafts (17) angeordnet sind;
die distale Ablationsanordnung (21) mindestens eine zweite Vielzahl von Elektroden (132) umfasst, die fest entlang der distalen Ablationsanordnung (21) angeordnet sind, wobei die distale Ablationsanordnung (21) so konfiguriert ist, dass sie einer vordefinierten Form entspricht, sodass die zweite Vielzahl von Elektroden (132) entsprechend der vordefinierten Form auf der distalen Ablationsanordnung (21) angeordnet sind, die sich über die Öffnung (30) aus dem distalen Abschnitt des länglichen Schafts (17) heraus erstreckt; und
alle Elektroden der mindestens ersten Vielzahl (127, 113 oder 114) und der zweiten Vielzahl (132) durch die Stromquelle (4) über ein elektrisches Signal (S) mit Strom versorgt werden, um nicht-thermische Energie zum Behandeln des Gewebes (41) und thermische Energie zum Abtragen des Gewebes (41) zu liefern;
wobei das elektrische Signal (S) eine Sinuswelle umfasst,
wobei das elektrische Signal (S) den Elektroden der ersten Vielzahl (127, 113 oder 114) und der zweiten Vielzahl (132) während eines Zeitintervalls (T) zugeführt wird, und
wobei das elektrische Signal (S) eine sinusförmige Impulsfolge (204) ist, die zwei oder mehr Basissinuswellen (BSW) in dem Zeitintervall (T) umfasst, wobei jede Basissinuswelle (BSW) aus einer positiven Halbwelle und einer negativen Halbwelle besteht.

2. Ablationsvorrichtung (100) nach Anspruch 1, wobei die Stromquelle eine einzelne Stromquelle ist.

3. Ablationsvorrichtung (100) nach Anspruch 1, wobei die Stromquelle (4) umfasst: eine einzige Steuereinheit (400) und eine Stromversorgungseinheit (401) zum Erzeugen des elektrischen Signals (S), das eine Sinuswelle umfasst, und
wobei die Stromversorgungseinheit (401) elektrisch mit allen Elektroden der ersten Vielzahl von Elektroden (127, 113 oder 114) und der zweiten Vielzahl von Elektroden (132) verbunden ist.

4. Ablationsvorrichtung (100) nach Anspruch 3, wobei die Steuereinheit (400) so konfiguriert ist, dass sie die Stromversorgungseinheit (401) ansteuert, um die Anzahl von Impulsen in der sinusförmigen Impulsfolge (204) zu modifizieren und so den mit dem elektrischen Signal (S) assoziierten elektrischen Energiepegel zu ändern.

5. Ablationsvorrichtung (100) nach Anspruch 3, wobei das sinusförmige Impulsfolgesignal (S) zwei bis fünfundzwanzig Basissinuswellen (BSW) in dem Zeitintervall (T) umfasst und/oder
wobei jede Basissinuswelle (BSW) eine Dauer umfasst, die einem ersten Zeitintervall (T1) entspricht, und/oder
wobei die Steuereinheit (400) so konfiguriert ist, dass sie die Stromversorgungseinheit (401) ansteuert, um die Dauer des ersten Zeitintervalls (T1) der Basissinuswelle (BSW) zu modifizieren, um den mit dem elektrischen Signal (S) assoziierten elektrischen Energiepegel zu ändern.

6. Ablationsvorrichtung (100) nach Anspruch 3, wobei das elektrische Signal (S), das eine Sinuswelle umfasst, ein Spannungssignal ist und eine mittlere Spitze-Spitze-Amplitude jeder Basissinuswelle (BSW) im Bereich von 1.000 V bis 2.000 V liegt.

7. Ablationsvorrichtung (100) nach Anspruch 3, wobei die Stromversorgungseinheit (401) ein Leistungsmodul (402) umfasst, das Folgendes umfasst:
- einen von der Steuereinheit (400) gesteuerten Ansteuerungsschaltungsblock (403) zum Erzeugen des elektrischen Signals (S), ausgehend von einem von der einzelnen Steuereinheit (400) bereitgestellten Versorgungsspannungssignal (Vcc);
- einen Auswahlblock (404), der selektiv von dem Ansteuerungsschaltungsblock (403) gesteuert wird, um den mit dem Signal (S) assoziierten elektrischen Energiepegel kontinuierlich zu ändern; und
- einen Block zur Filterung und elektrischen Isolierung (405, 406).

8. Ablationsvorrichtung (100) nach Anspruch 3, wobei die Steuereinheit (400) umfasst:
einen Mikroprozessor (407), der so konfiguriert ist, dass er einen variablen Hochspannungsversorgungsblock (408) und einen programmierbaren Logiksteuerungsblock (409) steuert; wobei
der variable Hochspannungsversorgungsblock (408) so konfiguriert ist, dass er das Versorgungsspannungssignal (Vcc) an das Leistungsmodul (402) bereitstellt, um das elektrische Signal (S) zu erzeugen;
der programmierbare Logiksteuerungsblock (409) so konfiguriert ist, dass er Ansteuersignale zum Steuern des Ansteuerungsschaltungsblocks (403) des Leistungsmoduls (402) erzeugt; und
die einzelne Steuereinheit (400) ferner umfasst:
- einen Video-Schnittstellen- und Drucktastenblock (410, 410'), der von dem Mikroprozessor (407) gesteuert wird, um Parameter der Vorrichtung (100) einzustellen und die ausgewählten Parameter anzuzeigen;
- einen Watchdog-Block (411) zum Steuern einer ordnungsgemäßen Funktion des Mikroprozessors (407); und
- einen Audio-Schnittstellenblock (412) zum Bereitstellen von Audioinformationen, die die Korrektheit des Ablationsprozesses und/oder aufgetretene Fehler anzeigen.

9. Ablationsvorrichtung (100) nach Anspruch 1, wobei
der Ablationskatheter (1) einen länglichen Katheterschaft (13) mit einer Längshauptrichtung (X-X) umfasst, wobei der längliche Katheterschaft (13) den distalen Abschnitt des länglichen Schafts (17) umfasst, wobei der distale Abschnitt des länglichen Schafts (17) ein distales Ende des distalen Abschnitts des Schafts (19) umfasst;
wobei der Ablationskatheter (1) ein inneres Lumen (118) umfasst, das innerhalb des länglichen Katheterschafts (13) angeordnet ist und das so konfiguriert ist, dass es die distale Ablationsanordnung (21) aufnehmen kann; wobei
der Ablationskatheter (1) Folgendes umfasst:
- die Schaft-Ablationsanordnung (20), die fest an dem distalen Abschnitt des Schafts (17) angeordnet ist, wobei die Schaft-Ablationsanordnung (20) so konfiguriert ist, dass sie sowohl thermische Energie zum Abtragen des Gewebes (41) als auch nicht-thermische Energie zum Behandeln des Gewebes (41) liefert; und
- mindestens einen Formgebungsdorn (26), der an der distalen Ablationsanordnung (21) so angeordnet ist, dass der Formgebungsdorn (26) innerhalb des Ablationskatheters (1) beweglich ist, wobei der Formgebungsdorn (26) in das innere Lumen (118) einführbar und aus dem inneren Lumen (118) entfernbar ist,
wobei der Formgebungsdorn (26) in Bezug auf das innere Lumen (118) frei beweglich ist, um während des Einführens des Formgebungsdorns jegliche Einschränkung durch den distalen Abschnitt des länglichen Schafts (17) zu vermeiden,
wobei der Formgebungsdorn (26) mindestens eine vorgeformte Konfiguration umfasst und der Formgebungsdorn (26) zwischen mindestens einer geraden verlaufenden Konfiguration und der vorgeformten Konfiguration reversibel verformbar ist,
wobei, wenn der Formgebungsdorn (26) vollständig in den distalen Abschnitt des länglichen Schafts (17) eingeführt ist, der Formgebungsdorn (26) so konfiguriert ist, dass er den distalen Abschnitt des länglichen Schafts (17) mit der vorgeformten Konfiguration formt.

10. Ablationsvorrichtung (100) nach Anspruch 9, wobei der distale Abschnitt des länglichen Schafts (17) elastisch verformbar ist und / oder wobei, wenn der Formgebungsdorn (26) vollständig in den distalen Abschnitt des länglichen Schafts (17) eingeführt ist, der distale Abschnitt des länglichen Schafts (17) so konfiguriert ist, dass er sich an die vorgeformte Konfiguration anpasst.

11. Ablationsvorrichtung (100) nach Anspruch 9, wobei, wenn der Formgebungsdorn (26) vollständig in den distalen Abschnitt des länglichen Schafts (17) eingeführt ist, eine vollständig eingeführte Position des Dorns definiert ist, und
wobei, während der Formgebungsdorn (26) innerhalb des inneren Lumens (118) in Richtung der vollständig eingeführten Position des Dorns gleitet, und wobei der Formgebungsdorn (26) so konfiguriert ist, dass er den distalen Abschnitt des länglichen Schafts (17), der von der gerade verlaufenden Konfiguration in die vorgeformte Konfiguration übergeht, variabel formt.

12. Ablationsvorrichtung (100) nach Anspruch 5, wobei das erste Zeitintervall (T1) ausgewählt ist im Bereich von 1 µsec bis 80.000 msec oder im Bereich von 75 µsec bis 20.000 msec oder im Bereich von 20 µsec bis 100 µsec.

13. Ablationsvorrichtung (100) nach Anspruch 1, wobei das sinusförmige Impulsfolge-Elektrosignal (S) den Elektroden (127, 113 oder 114) während eines Zeitintervalls (T), ausgewählt in dem Bereich von 100 µsec bis 100 sec, zugeführt wird.

14. Ablationsvorrichtung (100) nach Anspruch 1, wobei die Elektroden der mindestens ersten Vielzahl (127, 113 oder 114) von der Stromquelle (4) elektrisch gespeist werden, um eine Spannung zur Behandlung der Zielbereiche von Gewebe (41) zu liefern, die ausgewählt wird im Bereich von 100 V/cm bis 7.000 V/cm, oder im Bereich von 200 V/cm bis 2.000 V/cm oder im Bereich von 300 V/cm bis 1.000 V/cm.

15. Ablationsvorrichtung (100) nach Anspruch 1, wobei mindestens eine Elektrode der ersten Vielzahl von Elektroden (127) zwei voneinander elektrisch isolierte leitfähige Abschnitte (N) umfasst und / oder wobei mindestens eine Elektrode der ersten Vielzahl von Elektroden (127) vier voneinander elektrisch isolierte leitfähige Abschnitte (N) umfasst.

## Revendications

1. Équipement d'ablation (100) pour traiter des régions cibles de tissu (41) dans des organes (44), comprenant :
un cathéter d'ablation (1) et une source d'alimentation (4) ;
ledit cathéter d'ablation (1) comprenant :
une tige allongée de cathéter (13) comprenant au moins une partie distale de tige allongée (17) ;
ladite tige allongée de cathéter (13) comprenant un corps flexible (207) pour naviguer à travers des vaisseaux du corps (208) ;
ledit cathéter d'ablation (1) comprenant en outre un ensemble d'ablation à tige (20) disposé au niveau de ladite partie distale de tige allongée (17) et un ensemble d'ablation distal (21) extensible à travers ledit ensemble d'ablation à tige (20) et hors de ladite partie distale de tige allongée (17) via un orifice (30) dudit cathéter d'ablation (1) ;
ledit ensemble d'ablation à tige (2) comprenant au moins une première pluralité d'électrodes (127, 113 ou 114) disposée de manière fixe au niveau de ladite partie distale de tige allongée (17) ;
ledit ensemble d'ablation distal (21) comprenant au moins une deuxième pluralité d'électrodes (132) disposée de manière fixe le long dudit ensemble d'ablation distal (21), ledit ensemble d'ablation distal (21) étant configuré pour se conformer à une forme prédéfinie de telle sorte que la deuxième pluralité d'électrodes (132) est agencée selon la forme prédéfinie sur ledit ensemble d'ablation distal (21) s'étendant hors de ladite partie distale de tige allongée (17) via ledit orifice (30) ; et
toutes les électrodes desdites au moins la première pluralité (127, 113 ou 114) et la deuxième pluralité (132) étant alimentées électriquement par ladite source d'alimentation (4) à travers un signal électrique (S) pour délivrer de l'énergie non thermique pour le traitement du tissu (41) et de l'énergie thermique pour l'ablation du tissu (41) ;
dans lequel ledit signal électrique (S) comprend une onde sinusoïdale,
dans lequel ledit signal électrique (S) est fourni aux électrodes de ladite première pluralité (127, 113 ou 114) et de ladite deuxième pluralité (132) pendant un intervalle de temps (T), et
dans lequel ledit signal électrique (S) est un train d'impulsions sinusoïdales (204) comprenant deux ou plus ondes sinusoïdales de base (BSW) dans ledit intervalle de temps (T), dans lequel chaque onde sinusoïdale de base (BSW) est constituée d'une demi-onde positive et d'une demi-onde négative.

2. Équipement d'ablation (100) selon la revendication 1, dans lequel ladite source d'alimentation est une source d'alimentation unique.

3. Équipement d'ablation (100) selon la revendication 1, dans lequel ladite source d'alimentation (4) comprend une unité de commande unique (400) et une unité d'alimentation (401) pour la génération dudit signal électrique (S) comprenant une onde sinusoïdale, et
dans lequel ladite unité d'alimentation (401) est reliée électriquement à toutes les électrodes de ladite première pluralité d'électrodes (127, 113 ou 114) et de ladite deuxième pluralité d'électrodes (132).

4. Équipement d'ablation (100) selon la revendication 3, dans lequel ladite unité de commande (400) est configurée pour piloter l'unité d'alimentation (401) pour modifier le nombre d'impulsions dans le train d'impulsions sinusoïdales (204) pour changer le niveau d'énergie électrique associé au signal électrique (S).

5. Équipement d'ablation (100) selon la revendication 3, dans lequel ledit signal électrique (S) de train d'impulsions sinusoïdales comprend de deux à vingt-cinq ondes sinusoïdales de base (BSW) dans ledit intervalle de temps (T), et/ou
dans lequel chaque onde sinusoïdale de base (BSW) comprend une durée égale à un premier intervalle de temps (T1), et/ou
dans lequel ladite unité de commande (400) est configurée pour piloter l'unité d'alimentation (401) pour modifier la durée du premier intervalle de temps (T1) de l'onde sinusoïdale de base (BSW) pour changer le niveau d'énergie électrique associé au signal électrique (S).

6. Équipement d'ablation (100) selon la revendication 3, dans lequel ledit signal électrique (S) comprenant une onde sinusoïdale est un signal de tension, une amplitude moyenne de crête à crête de chaque onde sinusoïdale de base (BSW) est dans la plage de 1000 V à 2000 V.

7. Équipement d'ablation (100) selon la revendication 3, dans lequel ladite unité d'alimentation (401) comprend un module d'alimentation (402) comprenant :
- un bloc de circuit de pilotage (403) commandé par l'unité de commande (400) pour la génération dudit signal électrique (S) en commençant à partir d'un signal de tension d'alimentation (Vcc) fourni par l'unité de commande unique (400) ;
- un bloc de sélection (404) commandé de manière sélective par ledit bloc de circuit de pilotage (403) pour changer en continu le niveau d'énergie électrique associé audit signal (S) ; et
- un bloc de filtrage et d'isolation électrique (405, 406).

8. Équipement d'ablation (100) selon la revendication 3, dans lequel ladite unité de commande (400) comprend :
un microprocesseur (407) configuré pour commander un bloc d'alimentation haute tension variable (408) et un bloc de contrôleur logique programmable (409) ;
ledit bloc d'alimentation haute tension variable (408) étant configuré pour fournir ledit signal de tension d'alimentation (Vcc) au module d'alimentation (402) pour la génération dudit signal électrique (S) ;
ledit bloc de contrôleur logique programmable (409) étant configuré pour générer des signaux de pilotage pour commander le bloc de circuit de pilotage (403) du module d'alimentation (402) ; et
ladite unité de commande unique (400) comprenant en outre :
- un bloc d'interface vidéo et de bouton-poussoir (410, 410') commandé par le microprocesseur (407) pour régler des paramètres de l'équipement (100) et afficher les paramètres sélectionnés ;
- un bloc de chien de garde (411) pour la commande du bon fonctionnement du microprocesseur (407) ; et
- un bloc d'interface audio (412) pour la fourniture d'informations audio représentant la justesse du processus d'ablation et/ou des erreurs survenues.

9. Équipement d'ablation (100) selon la revendication 1, dans lequel
ledit cathéter d'ablation (1) comprenant ladite tige allongée de cathéter (13) ayant une direction principale longitudinale (X-X), ladite tige allongée de cathéter (13) comprenant ladite partie distale de tige allongée (17), ladite partie distale de tige allongée (17) comprenant une extrémité distale de partie distale de tige (19) ;
ledit cathéter d'ablation (1) comprenant une lumière interne (118) agencée à l'intérieur de la tige allongée de cathéter (13) qui est configurée pour recevoir l'ensemble d'ablation distal (21) ;
ledit cathéter d'ablation (1) comprenant :
- ledit ensemble d'ablation à tige (20) disposé de manière fixe au niveau de ladite partie distale de tige (17), l'ensemble d'ablation à tige (20) étant configuré pour délivrer à la fois de l'énergie thermique pour l'ablation dudit tissu (41) et de l'énergie non thermique pour le traitement dudit tissu (41) ; et
- au moins un mandrin de réglage de forme (26) disposé sur ledit ensemble d'ablation distal (21) de telle sorte que ledit mandrin de réglage de forme (26) est mobile à l'intérieur du cathéter d'ablation (1), le mandrin de réglage de forme (26) pouvant être inséré à l'intérieur de la lumière interne (118) et pouvant être retiré de la lumière interne (118),
dans lequel le mandrin de réglage de forme (26) est libre de se déplacer par rapport à la lumière interne (118) en évitant toute contrainte avec ladite partie distale de tige allongée (17) pendant l'insertion du mandrin de réglage de forme,
dans lequel le mandrin de réglage de forme (26) comprend au moins une configuration préformée et le mandrin de réglage de forme (26) est déformable de manière réversible entre au moins une configuration chargée droite et ladite configuration préformée,
dans lequel, lorsque le mandrin de réglage de forme (26) est entièrement inséré dans la partie distale de tige allongée (17), le mandrin de réglage de forme (26) est configuré pour régler la forme de ladite partie distale de tige allongée (17) avec ladite configuration préformée.

10. Équipement d'ablation (100) selon la revendication 9, dans lequel ladite partie distale de tige allongée (17) est élastiquement déformable et/ou dans lequel lorsque le mandrin de réglage de forme (26) est entièrement inséré dans la partie distale de tige allongée (17), ladite partie distale de tige allongée (17) est configurée pour se conformer à ladite configuration préformée.

11. Équipement d'ablation (100) selon la revendication 9, dans lequel lorsque le mandrin de réglage de forme (26) est entièrement inséré dans la partie distale de tige allongée (17), il est défini une position de mandrin entièrement insérée, et
dans lequel pendant que le mandrin de réglage de forme (26) coulisse à l'intérieur de la lumière interne (118) vers ladite position de mandrin entièrement insérée, et dans lequel le mandrin de réglage de forme (26) est configuré pour régler de manière variable la forme de la partie distale de tige allongée (17) passant de ladite configuration droite chargée à ladite configuration préformée.

12. Équipement d'ablation (100) selon la revendication 5, dans lequel ledit premier intervalle de temps (T1) est sélectionné dans la plage de 1 µs à 80 000 ms, ou sélectionné dans la plage de 75 µs-20 000 ms, ou sélectionné dans la plage de 20 µs-100 µs.

13. Équipement d'ablation (100) selon la revendication 1, dans lequel le signal électrique (S) de train d'impulsions sinusoïdales est fourni aux électrodes (127, 113 ou 114) pendant un intervalle de temps (T) sélectionné dans la plage de 100 µs-100 s.

14. Équipement d'ablation (100) selon la revendication 1, dans lequel les électrodes de ladite au moins la première pluralité (127, 113 ou 114) sont alimentées électriquement par ladite source d'alimentation (4) pour délivrer une tension pour traiter les régions cibles de tissu (41) laquelle est sélectionnée dans la plage de 100 V/cm-7000 V/cm, ou sélectionnée dans la plage de 200 V/cm-2000 V/cm, ou sélectionnée dans la plage de 300 V/cm-1000 V/cm.

15. Équipement d'ablation (100) selon la revendication 1, dans lequel au moins une électrode de ladite première pluralité d'électrodes (127) comprend deux parties conductrices (N) isolées électriquement l'une de l'autre et/ou dans lequel au moins une électrode de ladite première pluralité d'électrodes (127) comprend quatre parties conductrices (N) isolées électriquement les unes des autres.
